# EUROPÄISCHE PATENTSCHRIFT

(11) **EP 1 214 418 B1**
(45) Veröffentlichungstag und Bekanntmachung des Hinweises auf die Patenterteilung: **05.03.2008**
(21) Anmeldenummer: 00960626.0
(22) Anmeldetag: 08.09.2000
(51) Int. Cl.: C12N 15/52, C07K 14/44, C12N 9/02, C12N 1/10, C07K 16/20

(54) **NUKLEINSÄURE AUS TETRAHYMENA KODIEREND FUER EINE DELTA 6-DESATURASE, IHRE HERSTELLUNG UND VERWENDUNG**
NUCLEIC ACID WHICH IS OBTAINED FROM TETRAHYMENA AND WHICH CODES FOR A DELTA-6-DESATURASE, THE PRODUCTION THEREOF AND USE
ACIDE NUCLEIQUE OBTENU A PARTIR DE TETRAHYMENA, CODANT POUR UNE DELTA-6-DESATURASE, PREPARATION ET UTILISATION DUDIT ACIDE NUCLEIQUE

(30) Priorität: 10.09.1999 DE 19943270
(43) Veröffentlichungstag der Anmeldung: 19.06.2002
(73) Patentinhaber: Nutrinova Nutrition Specialties & Food Ingredients GmbH, 65926 Frankfurt am Main (DE)
(72) Erfinder: RÜSING, Matthias, 50935 Köln (DE); KIY, Thomas, D-65929 Frankfurt am Main (DE); DOMINITZKI, Annette, 55270 Klein-Winternheim (DE)
(74) Vertreter: Luderschmidt, Schüler & Partner
(86) Internationale Anmeldenummer: PCT/EP2000/008778
(87) Internationale Veröffentlichungsnummer: WO 2001/020000

(56) Entgegenhaltungen:
- BERTRAM J ET AL: "ISOLATION OF A STEAROYL COENZYME DESATURASE FROM TETRAHYMENA-THERMOPHILA" JOURNAL OF PROTOZOOLOGY, Bd. 28, Nr. 1, 1981, Seiten 127-131, XP002152567 ISSN: 0022-3921
- KOLL M ET AL: "THE EFFECT OF DIETARY STEROL ON THE ACTIVITY OF FATTY ACID DESATURASES ISOLATED FROM TETRAHYMENA-SETOSA" JOURNAL OF PROTOZOOLOGY, Bd. 37, Nr. 3, 1990, Seiten 229-237, XP002152568 ISSN: 0022-3921
- FUJIWARA Y ET AL: "CYTOPLASMIC LOCATION OF LINOLEOYL-COENZYME DESATURASE IN MICROSOMAL MEMBRANES OF RAT LIVER" ARCHIVES OF BIOCHEMISTRY AND BIOPHYSICS, Bd. 233, Nr. 2, 1984, Seiten 402-407, XP002152569 ISSN: 0003-9861
- NAPIER ET AL: "Identification of a Caenorhabditis elegans delta6-fatty-acid-desaturase by heterologos expression in Saccharomyces cerevisiae" BIOCHEMICAL JOURNAL,GB,PORTLAND PRESS, LONDON, Bd. 330, Nr. 2, März 1998 (1998-03), Seiten 611-614, XP002099453 ISSN: 0264-6021 & DATABASE GENBANK [Online] Accession No. AF031477, 2. Mai 1998 (1998-05-02) "Caenorhabditis elegans delta6-fatty-acid-desaturase mRNA, complete cds"

### SEQUENZ PROTOKOLL

<110> Aventis Research & Technologies GmbH & Co KG
<120> Neue Nukleinsäure aus Tetrahymena kodierend für eine delta 6-Desaturase, ihre Herstellung und Verwendung
<130> ax99046wo
<140>
   <141>
<150> DE 19943270.8
   <151> 1999-09-10
<160> 19
<170> Patent In Ver. 2.1
<210> 1
   <211> 1219
   <212> DNA
   <213> Tetrahymena thermophila
<400> 1
   agtaagcaaa ctaaatttaa aaaacaagca ttatgggagt tgataagact taagaagaaa 60
   ttgttcttga aaataaaccc gaacttctca acgaatacaa atttatttac aaggatactg 120
   aatatgactg cactgaatat gctaaatcaa ataagcatcc tggcggtctt aatttcctca 180
   atttgtttat tgatgagaag taagatttga ctgaatattt cagaacactc cattctaagt 240
   aggctttgaa aattttaaaa tccttcccta agactggcgc aaaataagag gagactgaat 300
   cttcaaagag attctcaata ttaaagaaaa agcttaagca tttattcgaa ccaaactggc 360
   ctatcgaaat tggtttattc ttaactacct ttactttatt tgtcactgga tgtttgactc 420
   aaaagtggta tttctctatt ccccttcttg tcttaatgca aatcatcagt ggttggattg 480
   gtcactctat gaaccacaat cgtaacccta tattaagaaa attcgcttta gtctacgctc 540
   ctctttgtgg tggtttctct aataaatggt ggggtaggaa gcacaatcaa catcatatgt 600
   tcacaaacaa cattctaaag gacgaagata tctaacacga ttacaaattg tggtaattcc 660
   ccttcttatt tttaaagtgg aaattagact ccatcttagc ttcttattat gaatttgaag 720
   gaatcttcct tgccttgcac tgggtattat tattcaacta aaacttctat atcgtaattc 780
   tttctgaatt gattgctggt ttcttcagtg cttctattct tgttggaaat catgaaaatg 840
   aaatgaaatt cgaaagaaga atcactttac catttttcga acatcaaata gctgcaagca 900
   gaaactacgc tttccacgac atattctctc tacttattat gggtggtatg taatattaga 960
   ctgaacatca ctttttccca taaattcctt tctacagatt acccaaagct cgtgtcataa 1020
   ttgctgaaga attaaagaag tggaacctta agattcatga aggacctatt tttgaaaaat 1080
   ctcacctttg aaaataaata aatttatttt aaatgcatat tttattagta atactaacaa 1140
   ttgtaggaaa tgtgttatgg tttgtttact tattactttt taatctgaga aaacagtctt 1200
   aacaaaaaaa aaaaaaaaa                                             1219
<210> 2
   <211> 352
   <212> PRT
   <213> Tetrahymena thermophila
<400> 2
   Met Gly Val Asp Lys Thr Gln Glu Glu Ile Val Leu Glu Asn Lys Pro
    1               5                  10                  15
   Glu Leu Leu Asn Glu Tyr Lys Phe Ile Tyr Lys Asp Thr Glu Tyr Asp
               20                  25                  30
   Cys Thr Glu Tyr Ala Lys Ser Asn Lys His Pro Gly Gly Leu Asn Phe
           35                  40                  45
   Leu Asn Leu Phe Ile Asp Glu Lys Gln Asp Leu Thr Glu Tyr Phe Arg
       50                  55                  60
   Thr Leu His Ser Lys Gln Ala Leu Lys Ile Leu Lys Ser Phe Pro Lys
   65                  70                  75                  80
   Thr Gly Ala Lys Gln Glu Glu Thr Glu Ser Ser Lys Arg Phe Ser Ile
                   85                  90                  95
   Leu Lys Lys Lys Leu Lys His Leu Phe Glu Pro Asn Trp Pro Ile Glu
               100                 105                 110
   Ile Gly Leu Phe Leu Thr Thr Phe Thr Leu Phe Val Thr Gly Cys Leu
           115                 120                 125
   Thr Gln Lys Trp Tyr Phe Ser Ile Pro Leu Leu Val Leu Met Gln Ile
       130                 135                 140
   Ile Ser Gly Trp Ile Gly His Ser Met Asn His Asn Arg Asn Pro Ile
   145                 150                 155                 160
   Leu Arg Lys Phe Ala Leu Val Tyr Ala Pro Leu Cys Gly Gly Phe Ser
                   165                 170                 175
   Asn Lys Trp Trp Gly Arg Lys His Asn Gln His His Met Phe Thr Asn
               180                 185                 190
   Asn Ile Leu Lys Asp Glu Asp Ile Gln His Asp Tyr Lys Leu Trp Gln
           195                 200                 205
   Phe Pro Phe Leu Phe Leu Lys Trp Lys Leu Asp Ser Ile Leu Ala Ser
       210                 215                 220
   Tyr Tyr Glu Phe Glu Gly Ile Phe Leu Ala Leu His Trp Val Leu Leu
   225 230 235 240
   Phe Asn Gln Asn Phe Tyr Ile Val Ile Leu Ser Glu Leu Ile Ala Gly
   245 250 255
   Phe Phe Ser Ala Ser Ile Leu Val Gly Asn His Glu Asn Glu Met Lys
   260 265 270
   Phe Glu Arg Arg Ile Thr Leu Pro Phe Phe Glu His Gln Ile Ala Ala
   275 280 285
   Ser Arg Asn Tyr Ala Phe His Asp Ile Phe Ser Leu Leu Ile Met Gly
   290 295 300
   Gly Met Gln Tyr Gln Thr Glu His His Phe Phe Pro Gln Ile Pro Phe
   305 310 315 320
   Tyr Arg Leu Pro Lys Ala Arg Val Ile Ile Ala Glu Glu Leu Lys Lys
   325 330 335
   Trp Asn Leu Lys Ile His Glu Gly Pro Ile Phe Glu Lys Ser His Leu
   340 345 350
<210> 3
   <211> 2492
   <212> DNA
   <213> Tetrahymena thermophila
<400> 3
   taaaacgatt ataaatatca cacaaattaa accgaaaaag agttaaagtg ctaatattaa 60
   taatataatt tatctaaatt gaaagatggt tcaattaatt tgaaattatt ttgaagcaaa 120
   ataattcgat tcgtgtaaga tggaaattga aagaattaag gtttagaaaa gttctttttg 180
   taaaataata gagttaaagt caataaattt tatattacgt aaatcttaaa gtgtgcaaat 240
   gttatcatta acaattctaa atgatgcaaa atatttaaat tattaaaaat aatgatagtt 300
   aataaaatca atatttcata ataataataa ggtatctatc tatctatcaa tatttcaata 360
   aatattaatt aaaaggttat aaaataagta agcaaactaa atttaaaaaa caagcattat 420
   gggagttgat aagacttaag aagaaattgt tcttgaaaat aaacccgaac ttctcaacga 480
   atacaaattt atttacaagg atactgaata tgactgcact gaatatgcta aatcaaataa 540
   gcatcctggc ggtcttaatt tcctcaattt gtttattgat gagaagtaag atttgactga 600
   atatttcaga acactccatt ctaagtaggc tttgaaaatt ttaaaatcct tccctaagac 660
   tggcgcaaaa taagaggaga ctgaatcttc aaagagattc tcaatattaa agaaaaagct 720
   taagcatgta aatacattca aatgatatct ttattgagca tatttagcat aatttgataa 780
   ttttcataag catattttaa attataaaaa tgaacatatt tttaaattaa tttagttatt 840
   cgaaccaaac tggcctatcg aaattggttt attcttaact acctttactt tatttgtcac 900
   tggatgtttg actcaaaagt ggtatttctc tattcccctt cttgtcttaa tgcaaatcat 960
   cagtggttgg attggtcact ctatgaacca caatcgtaac cctatattaa gaaaattcgc 1020
   tttagtctac gctcctcttt gtggtggttt ctctaataaa tggtggggta ggaagcacaa 1080
   tcaagtaacc ataatattta atataaatat ataaagattt tttggttttg cgaggaaaaa 1140
   agtcatattt tgatgcttta atagtacaaa caatatttga ttgttatgat taaattatta 1200
   aagatcttaa tttagccttt tttaaaaatt tcaaataaat ttgaagataa tattattaaa 1260
   gtataataaa tgattaagcc aaaatctgta ccaaaaatct gtaaatacaa aatcaacttc 1320
   acacaaagat tacacatagc attttatttt ttataataaa ataaatgaaa atagtttttt 1380
   attttaagaa atgaaataac tttttttccc tatgattttc aattaataaa aagcattgct 1440
   atacaaataa ttgaaaaaag ctaaatcttt tttctattaa aattaattac aaattgtaaa 1500
   agattaattt taccatttaa tttaagtacc gcaataagca aatctctatt ttttttaagc 1560
   aatgacgtca cggataaata ttatcatact attcctcaat aataaatcat ctttaaaata 1620
   atttaaaact aattaatata attctaataa aagcatcata tgttcacaaa caacattcta 1680
   aaggacgaag atatctaaca cgattacaaa ttgtggtaat tccccttctt atttttaaag 1740
   tggaaattag actccatctt agcttcttat tatgaatttg aaggaatctt ccttgccttg 1800
   cactgggtat tattattcaa ctaaaacttc tatatcgtaa ttctttctga attgattgct 1860
   ggtttcttca gtgcttctat tcttgttgga aatcatgaaa atgaaatgaa attcgaaaga 1920
   agaatcactt taccattttt cgaacatcaa atagctgcaa gcagaaacta cgctttccac 1980
   gacatattct ctctacttat tatgggtggt atgtaatatt agactgaaca tcactttttc 2040
   ccataaattc ctttctacag attacccaaa gctcgtgtca taattgctga agaattaaag 2100
   aagtggaacc ttaagattca tgaaggacct atttttgaaa aatctcacct ttgaaaataa 2160
   ataaatttat tttaaatgca tattttatta gtaatactaa caattgtagg aaatgtgtta 2220
   tggtttgttt acttattact ttttaatctg agaaaacagt cttaacattt attcgatttt 2280
   atttaacatt actttttaaa aaacaatttt gcttactata aatttacata agtatagtaa 2340
   gaaactaagt tgatggtgtt attttttaat ttttctaatt aatttgtgaa taaacgatga 2400
   tttaatttat taatccagca aataggcata attatattac aaataccagc ccgggccgtc 2460
   gaccacgcgt gccctatagt gagtcgtatt ac                              2492
<210> 4
   <211> 10
   <212> PRT
   <213> Tetrahymena thermophila
<400> 4
   Trp Trp Lys Trp Asn His Asn Ala His His
   1               5                   10
<210> 5
   <211> 13
   <212> PRT
   <213> Tetrahymena thermophila
<400> 5
   Gly Gly Leu Gln Phe Gln Ile Glu His His Leu Phe Pro
   1 5 10
<210> 6
   <211> 20
   <212> DNA
   <213> Künstliche Sequenz
<220>
   <223> Beschreibung der künstlichen Sequenz:Primer
<400> 6
   tggtggaart ggamncayaa 20
<210> 7
   <211> 20
   <212> DNA
   <213> Künstliche Sequenz
<220>
   <223> Beschreibung der künstlichen Sequenz:Primer
<400> 7
   cgdggraana rrtgrtgttc 20
<210> 8
   <211> 40
   <212> DNA
   <213> Künstliche Sequenz
<220>
   <223> Beschreibung der künstlichen Sequenz:Primer
<400> 8
   gaccacgcgt atcgatgtcg actttttttt tttttttttv 40
<210> 9
   <211> 28
   <212> DNA
   <213> Künstliche Sequenz
<220>
   <223> Beschreibung der künstlichen Sequenz:Primer
<400> 9
   ggaatcacaa tcaacatcat atgttcac 28
<210> 10
   <211> 29
   <212> DNA
   <213> Künstliche Sequenz
<220>
   <223> Beschreibung der künstlichen Sequenz:Primer
<400> 10
   cttcgtcctt tagaatgttg tttgtgaac 29
<210> 11
   <211> 29
   <212> DNA
   <213> Künstliche Sequenz
<220>
   <223> Beschreibung der künstlichen Sequenz:Primer
<400> 11
   agtaagcaaa ctaaatttaa aaaacaagc 29
<210> 12
   <211> 29
   <212> DNA
   <213> Künstliche Sequenz
<220>
   <223> Beschreibung der künstlichen Sequenz:Primer
<400> 12
   agtaagcaaa ctaaatttaa aaaacaagc 29
<210> 13
   <211> 30
   <212> DNA
   <213> Künstliche Sequenz
<220>
   <223> Beschreibung der künstlichen Sequenz:Primer
<400> 13
   ggtccttcat gaatcttaag gttccacttc 30
<210> 14
   <211> 27
   <212> DNA
   <213> Künstliche Sequenz
<220>
   <223> Beschreibung der künstlichen Sequenz:Primer
<400> 14
   cttaagtctt atcaactccc ataatgc 27
<210> 15
   <211> 30
   <212> DNA
   <213> Künstliche Sequenz
<220>
   <223> Beschreibung der künstlichen Sequenz:Primer
<400> 15
   gaagtggaac cttaagattc atgaaggacc 30
<210> 16
   <211> 30
   <212> DNA
   <213> Künstliche Sequenz
<220>
   <223> Beschreibung der künstlichen Sequenz:Primer
<400> 16
   gcattatgca tgttgataag acttaagaag 30
<210> 17
   <211> 35
   <212> DNA
   <213> Künstliche Sequenz
<220>
   <223> Beschreibung der künstlichen Sequenz:Primer
<400> 17
   tatggatcct caaaggtgag atttttcaaa aatag 35
<210> 18
   <211> 30
   <212> DNA
   <213> Künstliche Sequenz
<220>
   <223> Beschreibung der künstlichen Sequenz:Primer
<400> 18
   aaaaataaaa aagtttgaaa aaaaaccttc 30
<210> 19
   <211> 22
   <212> DNA
   <213> Künstliche Sequenz
<220>
   <223> Beschreibung der künstlichen Sequenz:Primer
<400> 19
   gtttagctga ccgattcagt tc 22

## Beschreibung

Die vorliegende Erfindung betrifft eine delta-6-Desaturase aus Tetrahymena, ihre kodierende Nukleinsäure sowie ihre Herstellung und Verwendung.

Die Erfindung betrifft eine Nukleinsäure aus Tetrahymena die für eine ciliatenspezifische delta-6-Desaturase kodiert, welche an der Biosynthese von kommerziell wertvollen mehrfach ungesättigten Fettsäuren (sog. PUFA engi.: polyunsatturated fatty acids) in Eukaryoten beteiligt ist. Die erfindungsgemäßen Nukleinsäuren und die daraus erhältlichen Polypeptide zeigen dabei eine überraschend geringe Sequenzidentität zu anderen bekannten natürlichen Desaturasen. Die Erfindung betrifft ferner die Verwendung der Nukleinsäuren zur Überexpression in Eukaryoten insbesondere Ciliaten, vorzugsweise Tetrahymena, besonders bevorzugt *Tetrahymena thermophila,* mit dem Ziel der gezielten Modifikation des Fettsäurespektrums, insbesondere der Steigerung der PUFA-Bildung.

Die erfindungsgemäßen Nukleinsäuren sind erhältlich aus Ciliaten, vorzugsweise aus Tetrahymena, besonders bevorzugt aus *Tetrahymena thermophila,* einem GLA produzierenden Organismus mit sehr hohem GLA-Gehalt.

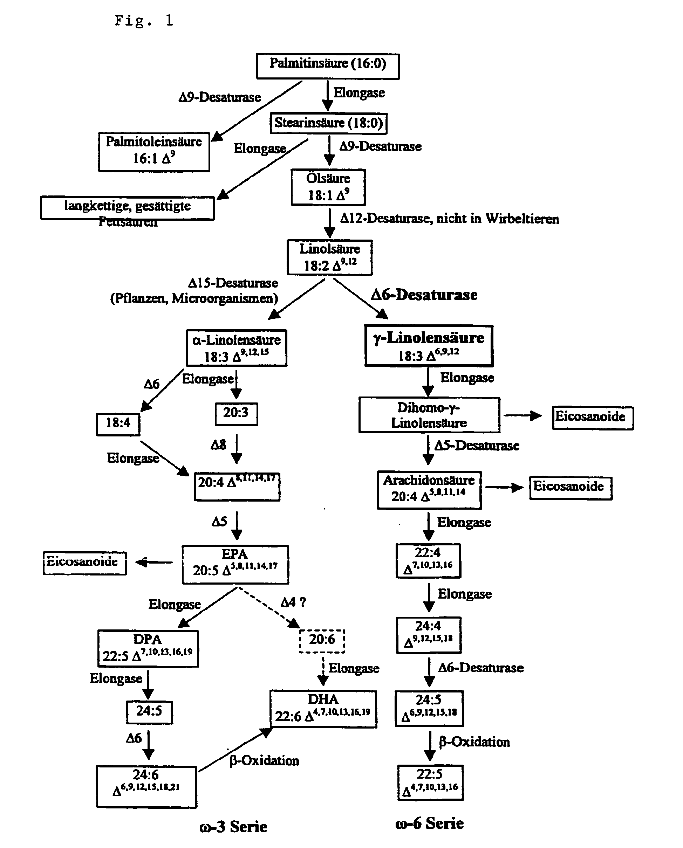

In Figur 1 ist ein allgemeines Schema der Biosynthese von PUFAs und den beteiligten Enzymen in Eukaryoten (modifiziert nach Gill & Valivety, Trends Biotechnol. 1997, 15:401-409) dargestellt. Die Umwandlung von Stearinsäure (18:0) zu Ölsäure (18:1 Δ9) wird durch eine delta 9-Desaturase katalysiert. Ölsäure wird durch eine delta 12-Desaturase in Linolsäure (18:2 Δ9, 12; kurz LA) umgewandelt, die wiederum durch eine delta-6-Desaturase in γ-Linolensäure (18:3, Δ6,9,12; kurz: GLA), bzw. durch eine delta 15-Desaturase in α-Linolensäure (18:3 Δ9,12,15; kurz: ALA) umgewandelt wird. Die Verlängerung der Fettsäuren wird durch Elongasen katalysiert, wodurch z. B. aus γ-Linolensäure Dihomo-γ-Linolensäure (20:3 Δ8,11,15; kurz: DGLA) gebildet wird, die wiederum durch eine delta 5-Desaturase zu Arachidonsäure (20:4 Δ5,8,11,15; kurz: ARA) umgewandelt wird, einem direkten Vorläufer physiologisch wirksamer Eicosanoide, wie z. B. Prostaglandine, Prostacycline, Thromboxane, Leukotriene. Bei der Bildung der PUFAs, die sich von GLA ableiten (nachfolgend delta 6-ungesättigte Fettsäuren genannt), hat sich die Umwandlung von LA zu GLA durch die delta-6-Desaturase als limitierender Schritt gezeigt (Huang YS & Mills DE (1996) γ-linolenic acid. Metabolism and its role in nutrition and medicin. AOCS Press, Champaign, Illinois, 1996).

Das Vorhandensein eines Enzymes mit delta-6-Desaturase Aktivität in Tetrahymena setosa und T. pyriformis ist bekannt (Peng, Y.M. und Elson, C.E. (1971) J. Nutr. 101, 1177-1184), aber ein homogenes Protein mit einer solchen Aktivität aus einem Ciliaten wurde bisher nicht zur Verfügung gestellt (z.B. Koll, M. und Erwin, J.A. (1990) J. Protozool. 37(3), 229-237).

Da Vertebraten keine Doppelbindungen hinter Position 9 in Fettsäuren einfügen können, sind ungesättigte Fettsäuren wie LA und ALA essentielle Nährstoffe, die von Vertebraten nicht synthetisiert werden können (siehe Figur 1), und in der Ernährung hauptsächlich aus pflanzlichen Quellen stammen. LA kann von Säugern durch eine delta-6-Desaturase in GLA umgewandelt werden - eine ARA-Vorstufe - die ein essentieller Vorläufer der meisten Prostaglandine ist. Die Bildung von Stearidonsäure (18:4 Δ6,9,12,15) - einem Vorläufer der EPA - aus ALA wird ebenfalls mittels delta-6-Desaturase katalysiert. Die delta-6-Desaturase ist damit der erste essentielle Schritt in der Biosynthese der Eicosanoide (siehe Figur 1).

Es hat sich gezeigt, daß die Aktivität der delta-6-Desaturase bei Säugern durch Faktoren wie z. B. Alkoholkonsum, Streß, Mangelemährung und Alterungsprozesse beeinträchtigt werden kann (Huang & Mills, 1996; Horrobin (1990) Rev. Contemp. Pharmacother. 1: 1-45; Bolton-Smith C et al. (1997) Eur. J. Clin. Nutr. 51:619-624; Leventhal LJ et al. (1993) Ann. Intern. Med. 119:867-873). Dies führt zu einer Unterversorgung mit GLA und damit letztlich zu einem Mangel der aus GLA abgeleiteten Moleküle wie ARA und der daraus gebildeten physiologisch wichtigen Eicosanoide, da es sich wie bereits erwähnt bei der Bildung der GLA aus LA durch die delta-6-Desaturase um einen limitierenden Schritt der PUFA-Synthese handelt (Brenner RR (1976) Adv. Exp. Med. Biol. 83:85-101; Nakahara T et al. (1993) J. Jpn. Oil Chem. Soc. 42:242-253; Chapkin, RS (1998) Reappraisal of the essential fatty acids. In: Fatty acids in food and their health implications, 2nd ed. (Chow CK, ed) Marcel Dekker, New York, NY). Die Zuführung von GLA kann sowohl einen reduzierten endogenen Spiegel von delta-6 ungesättigten Fettsäuren ausgleichen, als auch einen erhöhten Bedarf an diesen Fettsäuren decken (Horrobin (1990)). Für die Biosynthese von aus GLA abgeleiteten Molekülen ist deshalb die Aufnahme von GLA durch die Nahrung vorteilhaft (Fan, YY & Chapkin, RS (1998) J. Nutr. 128:1411-1414).

Der Befund, daß GLA vielfältige positive Einflüsse auf den menschlichen Körper ausübt, wurde inzwischen durch eine Vielzahl wissenschaftlicher Studien untermauert. So wurde die positive Wirkung der GLA z. B. auf atopische Ekzeme (Shimasaki, H: PUFA content and effect of dietary intake of γ-linolenic acid-rich oil on profiles of n-6, n-3 metabolites in plasma of children with atopic eczema. J. Clin. Biochem. Nutr. (1995), 19(3), 183-192.), Rheumatische Arthritis (Zurier RB, Rossetti RG, Jacobson EW, DeMarco DM, Liu NY, Temming JE, White BM, Laposata M (1996) γ-linolenic acid treatment of rheumatoid arthritis: A randomized, placebo-controlled trial. Arthritis Rheum. 39(11) 1808-1817), Atheriosklerose (Leng GC, Lee AJ, Fowkes FGR, Jepson RG, Lowe GDO, Skinner ER, Mowat BF, Randomized controlled trial of γ-linolenic acid and eicosapentaenoic acid in peripheral arterial disease, Clinical Nutrition, (1998) 17/6 265-271.), diabetische Neuropathie (Pfeifer MA, Schumer MP (1995) Clinical trials of diabetic neuropathy: past, present, and future. Diabetis 44(12) 1355-61), Migräne (Wagner W, Nootbaar-Wagner U (1997) Prophylactic treatment of migraine with γ-linolenic and alphalinolenic acids, Cephalalgia 17/2 127-130), Schizophrenie (Vaddadi, KS (1982) Some observations on the use of prostaglandin E1 precursor in the treatment of schizophrenia. Biol. Aspects Schizophr. Addict. 183-91. Publisher: Wiley, Chichester, UK) und Krebs (Kairemo KJA, Jekunen AP, Korppi-Tommola ET, Pyrhonen SO (1997) Effects of lithium γ-linolenate on the perfusion of liver and pancreatic tissues in pancreatic cancer. Anticancer Research 17/5 B 3729-3736.) durch klinische Studien nachgewiesen. In diesen Studien wurde sowohl eine statistisch als auch eine klinisch signifikante Verbesserung des Krankheitsbildes erzielt. Die Wirkung der GLA beruht dabei vor allem auf der Bildung von

Eicosanoiden (Prostaglandine, Prostacycline, Thromboxane, Leukotriene), für die GLA ein Vorläufermolekül in der Biosynthese darstellt (Figur 1).

Aufgrund dieser positiven Eigenschaften besteht ein breites Anwendungsspektrum für GLA in der Pharma-, Kosmetik-, Tierfutter- und Lebensmittelindustrie (Horrobin (1990), Horrobin (1992) Prog. Lipid Res. 31:163-194; Chapkin (1998), Fan & Chapkin (1998).

Die meisten PUFAs aus Menschen und Tieren stammen entweder direkt aus der Ernährung oder entstehen durch die Umsetzung der durch die Ernährung zugeführten essentiellen Fettsäuren durch Desaturasen und Elongasen. Deshalb sind die Gene der PUFA-Biosynthese aus Organismen, in denen diese PUFAs natürlicherweise vorkommen, von großem kommerziellen Interesse. Durch die gezielte, funktionelle Expression dieser Gene in Organismen oder Zellen kann eine kommerzielle Produktion von PUFAs in diesen Systemen erreicht werden. Aus diesem Grund besteht ein Bedarf an Genen für Desaturasen und Elongasen der PUFA-Biosynthese, sowie für die kommerzielle Gewinnung von PUFAs und PUFA-Ölen durch zuverlässige ökonomische Methoden mit Hilfe dieser Gene.

Keine der kommerziell genutzten Ölsaaten produziert GLA. GLA kommt dagegen nur im Öl der Samen verschiedener Pflanzen wie der Nachtkerze *(Oenothera biennis,* ca. 10% GLA), Borretsch *(Borago offcinalis,* ca. 23%) und der Schwarzen Johannisbeere *(Ribes nigrum,* ca. 18%) vor. Daneben sind auch verschiedene Mikroorganismen als Quellen für GLA bekannt, wie z. B. die Pilze *Mucor* und *Mortierella* (bis zu ca. 25%), die Blaualge *Spirulina* (ca. 12-18%) und andere. Als besonders GLA-reiche Quelle wurden Ciliaten wie z. B. *Tetrahymena* (bis zu 47%; Hill, DL (1972) The biochemistry and physiology of Tetrahymena. Chapter 3, 46-73. Academic press, New York, London; Erwin, J & Bloch, K (1963) J. Biol. Chem. 238:1618-1624) beschrieben. Eine gute Übersicht über natürliche Quellen von GLA geben Phillips & Huang (Phillips JC, Huang YS (1996) Natural sources and biosynthesis of γ-linolenic acid: an overview. 1-13 In: γ-linolenic acid. Metabolism and its role in Nutrition and medicin. Huang YS, Mills DE (eds.) AOCS Press, Champaign, Illinois, 1996).

Die kommerzielle Gewinnung von GLA aus diesen natürlichen Quellen ist jedoch mit einigen Nachteilen verbunden. Sowohl die Qualität als auch die Quantität der aus diesen Organismen gewonnen Öle schwanken, und teilweise weisen die Öle eine sehr heterogene Zusammensetzung auf, was aufwendige und teure Reinigungsschritte nötig macht um die GLA anzureichern. Der Anbau GLA-haltiger Pflanzen ist darüberhinaus nicht sehr wirtschaftlich (Hansen CE et al. (1991) J. Sci. Food Agric. 54:309-312). Für die Gewinnung von GLA-haltigem Öl hat sich gezeigt, daß die Raum/Zeit-Ausbeute bei einigen GLA-produzierenden Mikroorganismen im Vergleich zu höheren Pflanzen deutlich besser ist. Aus diesem Grunde bietet die fermentative Herstellung von GLA durch Mikroorganismen eine vielversprechende Alternative zu anderen GLA-Quellen. Das Fettsäurespektrum vieler Mikroorganismen ist oft recht einfach im Vergleich zu höheren Organismen, was grosse Vorteile bei der Reinigung bietet.. Darüber hinaus ist die fermentative Herstellung nicht von externen Faktoren wie Wetter, Nahrungsangebot etc. abhängig. Ausserdem sind auf diese Weise hergestellte PUFAs weitgehend frei von Kontaminationen die z.B. auf Umweltverschmutzung zurückzuführen sind. Ein weiterer Vorteil ist, dass durch fermentative Prozesse gewonnene GLA im Gegensatz zu GLA aus natürlichen Quellen keinen Schwankungen in der Verfügbarkeit unterliegen.

Es gab bereits Versuche, die fermentative Gewinnung von GLA zu etablieren (Ratledge C (1993) Trends Biochem. 11:278-284; Ratledge C (1989) Biochem. Soc. Trans. 17:1139-1141; Gosselin Y et al. (1989) Biotechnol. Lett. 11:423-426; WO86/03518). Für eine kommerzielle, fermentative GLA-Produktion durch Mikroorganismen ist aber eine Steigerung des GLA-Gehaltes wünschenswert, da die Fermentation PUFA-produzierender Mikroorganismen als relativ aufwendig und teuer und damit als nicht sehr wirtschaftlich erachtet wird (supra Ratledge 1993). Aufgrund seines relativ hohen GLA-Gehalts (supra) ist besonders *Tetrahymena thermophila* für eine fermentative Gewinnung von GLA geeignet. Tetrahymena kann gut im Fermenter kultiviert werden und es können hohe Zelldichten erreicht werden (Kiy, T. & Tiedtke (1992) Appl. Microbiol. Biotechnol. 37, 576-579; Kiy, T. & Tiedtke, A. (1992) Appl. Microbiol. Biotechnol. 38, 141-146).

Aufgabe der vorliegenden Erfindung ist es daher, Nukleinsäuren aus Tetrahymena bereitzustellen, welche für ein Polypeptid mit der Aktivität einer delta-6-Desaturase kodieren, und deren funktionelle Expression und Überexpression in einem Wirtsorganismus, vorzugsweise in Tetrahymena, zwecks Anreicherung von GLA und/oder delta 6-ungesättigten Fettsäuren.

Gegenstand der vorliegenden Erfindung ist daher eine Nukleinsäure gemäß SEQ ID No.: 1 kodierend für eine delta-6-Desaturase mit einer Aminosäuresequenz gemäß SEQ ID No.: 2 und Teile davon mit mindestens 8 Nukleotiden, vorzugsweise mit mindestens 15 oder 20 Nukleotiden, insbesondere mit mindestens 100 Nukleotiden, vor allem mit mindestens 300 Nukleotiden (nachfolgend "erfindungsgemäße Nukleinsäure(n)" genannt). Ein weiterer Gegenstand der Erfindung ist ebenfalls eine Nukleinsäure gemäß SEQ ID No.: 3, enthaltend die genomische Sequenz und die neben der kodierenden Sequenz für eine delta-6-Desaturase auch nicht kodierende Nukleinsäuresequenzen wie Introns, Promoter und flankierende Sequenzen, beinhaltet.

Die vollständige Nukleinsäure gemäß SEQ ID No.: 1 kodiert für ein Protein mit 352 Aminosäuren und einer theoretischen molekularen Masse von 41,8 kDa. Die Sequenzanalysen gemäß der vorliegenden Erfindung bestätigen, daß es sich bei der Nukleinsäure um eine Nukleinsäure handelt, die für eine delta-6-Desaturase aus Tetrahymena kodiert.

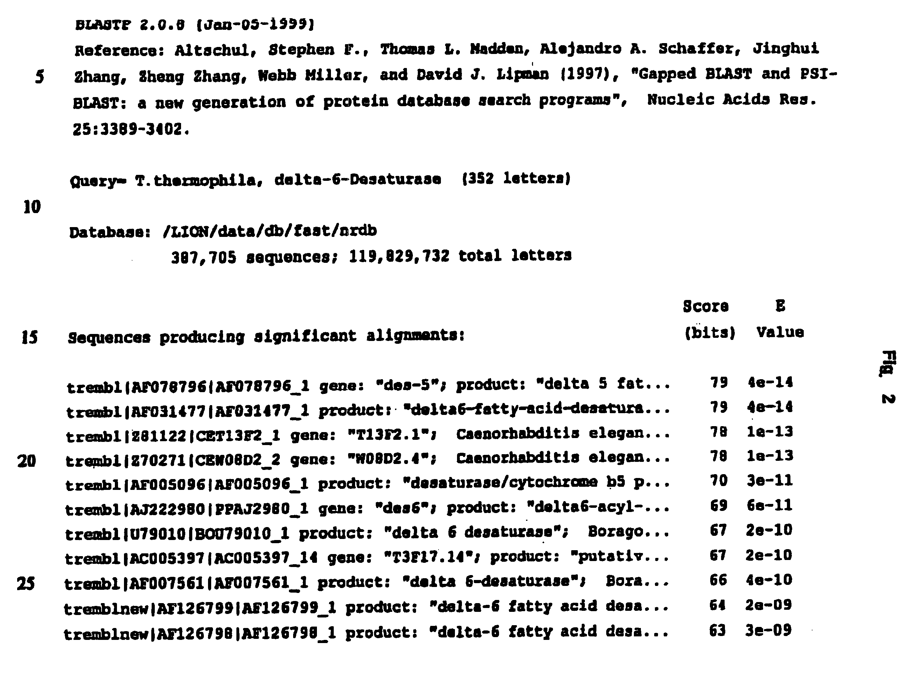

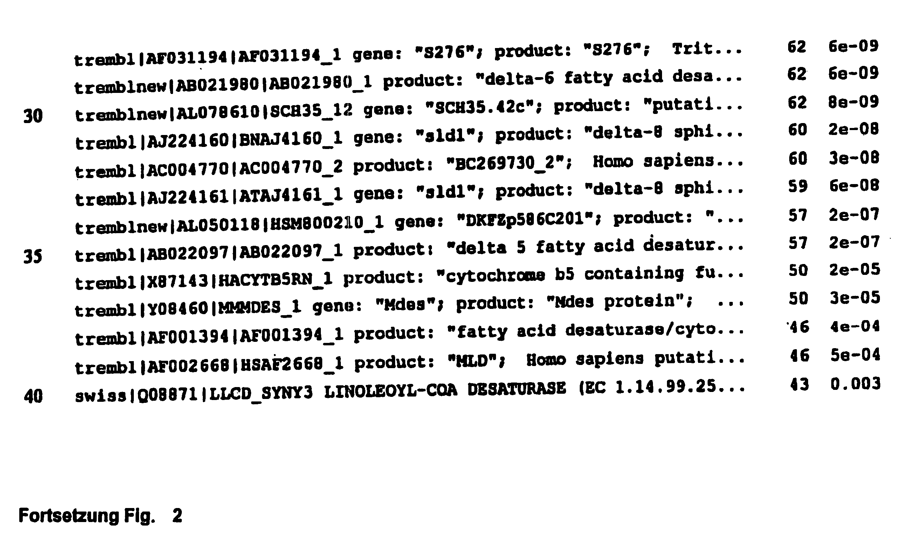

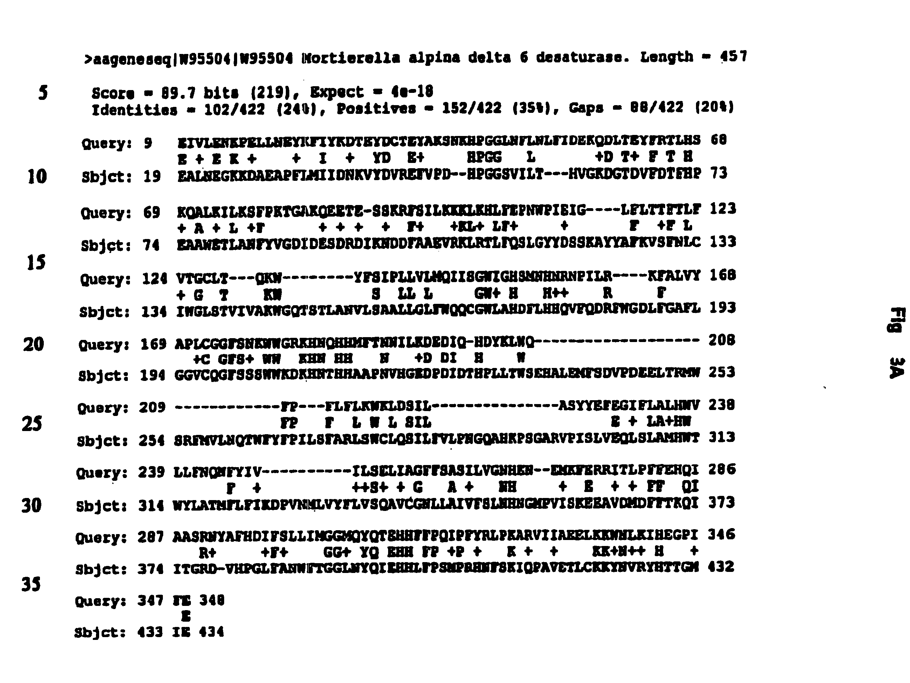

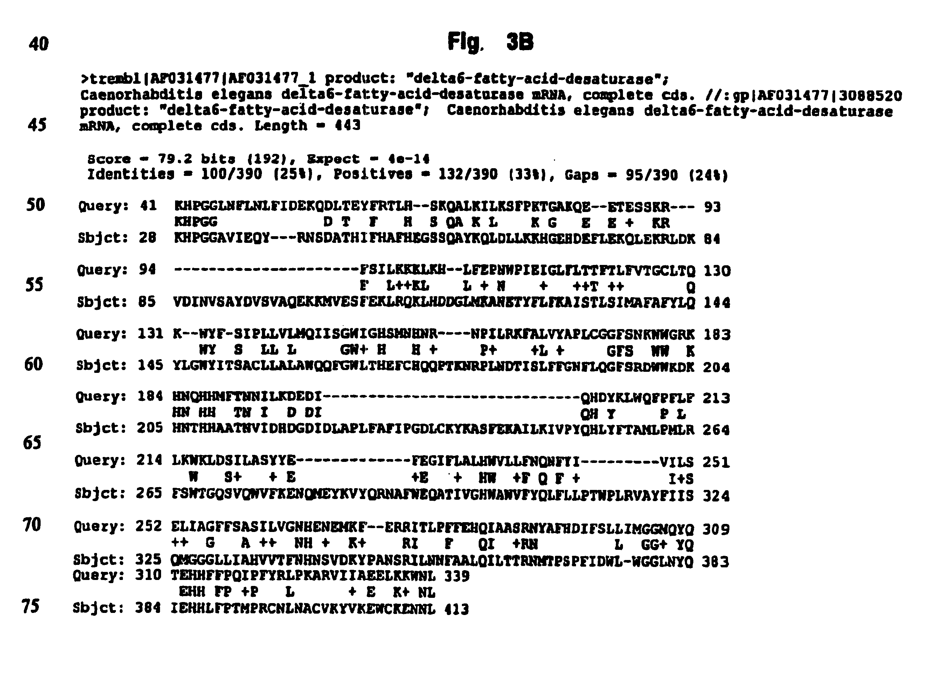

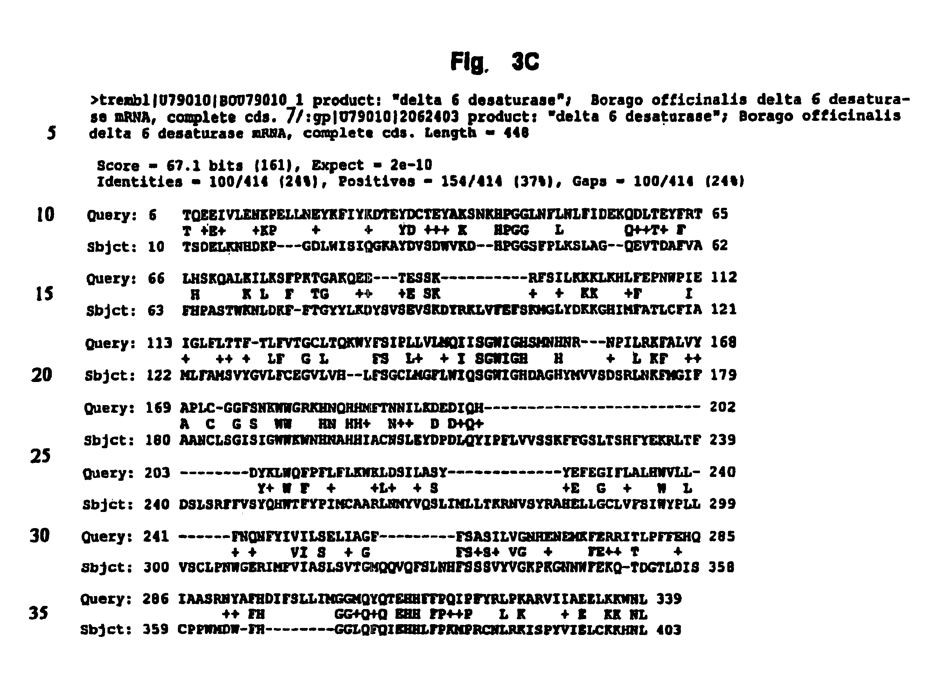

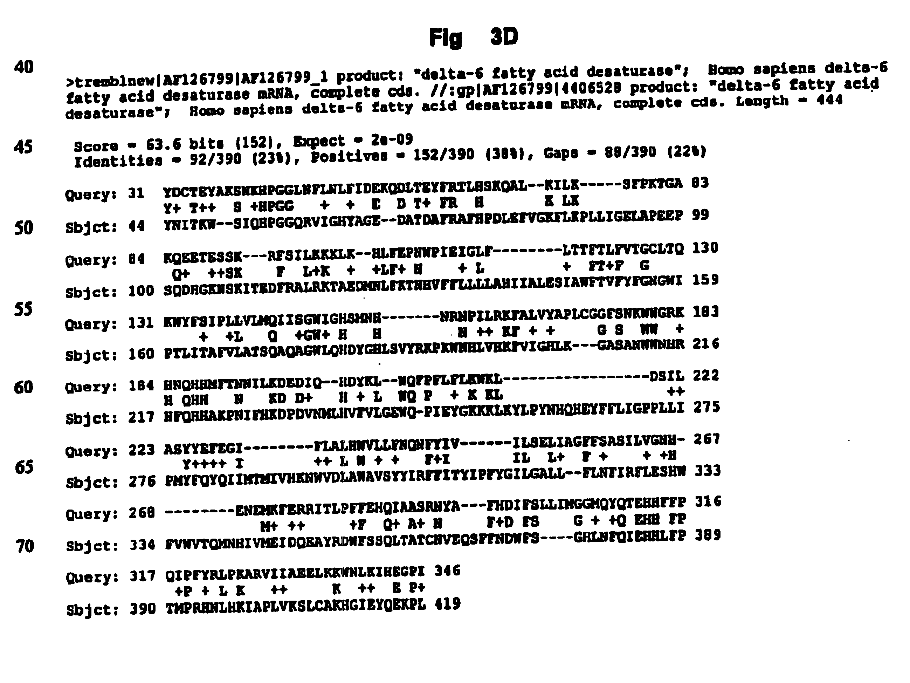

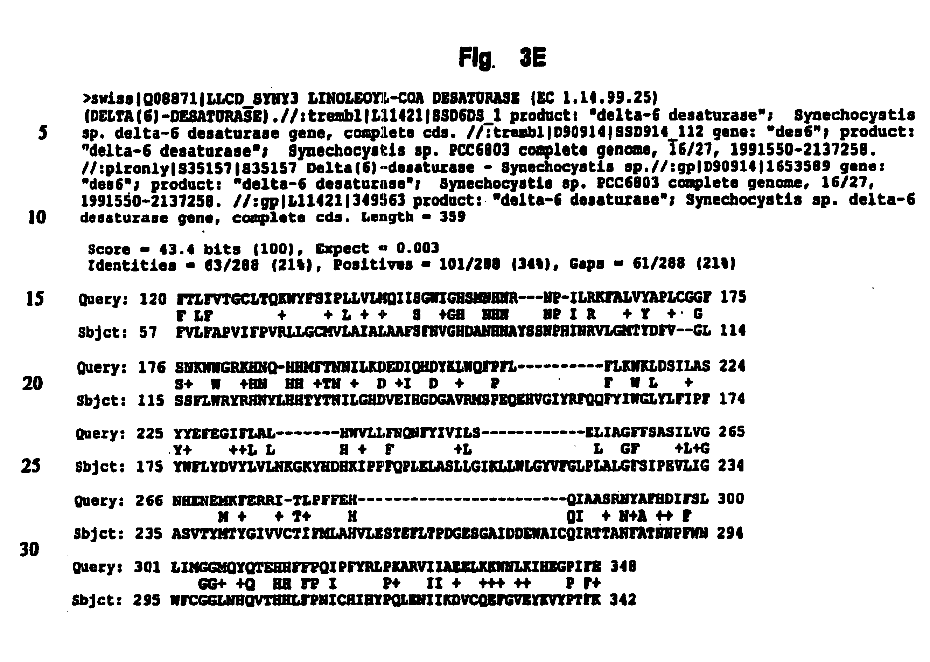

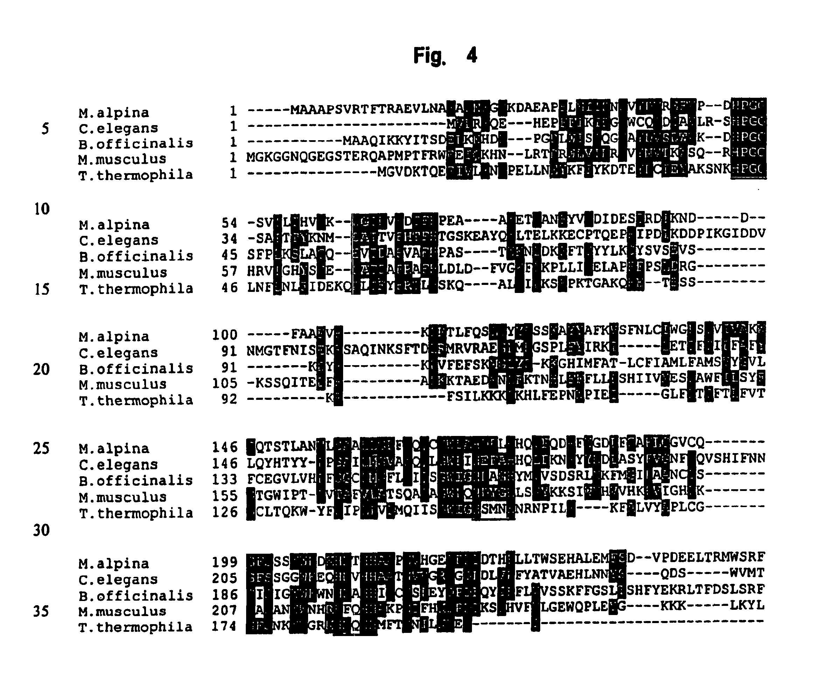

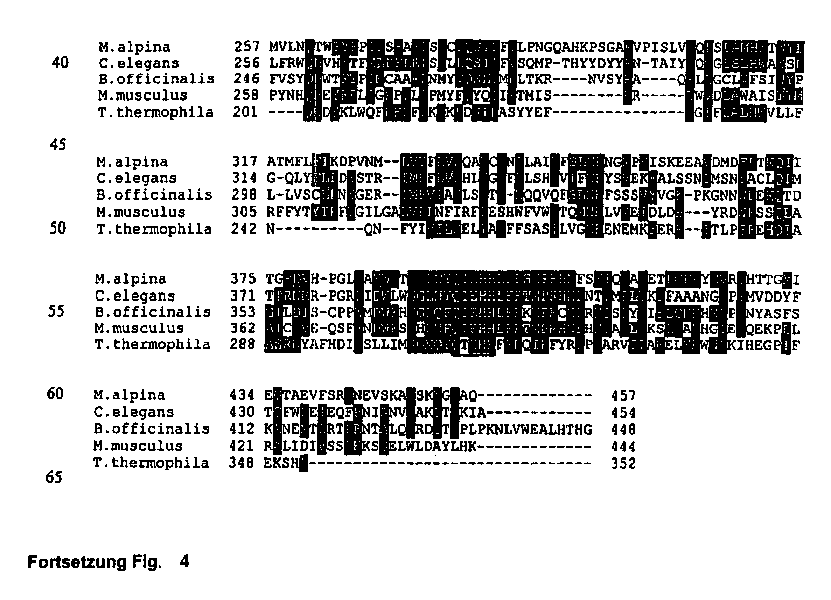

Durch einen Homologievergleich konnte die aus der Nukleinsäuresequenz (SEQ ID No.: 1) abgeleitete Proteinsequenz gemäß SEQ ID No.: 2 als eine delta-6-Desaturase identifiziert werden. Für den Homologievergleich wurde die Funktion BLASTP (Altschul et al. (1997) Nucleic Acids Res. 25:3389-3402) benutzt. Als homologe Proteine wurden Desaturasen, insbesondere delta-6-Desaturasen (E.C. 1.14.99.25; Linoleoyl-CoA desaturase) aus den Datenbanken identifiziert (siehe Figur 2). Die bekannten delta-6-Desaturasen weisen dabei maximal eine Identität von 25% zu der erfindungsgemäßen Polypeptidsequenz auf (siehe Figuren 3A-3E). Ein Multiples Alignment verschiedener bekannter delta-6-Desaturasen mit der erfindungsmäßen Polypeptidsequenz ist in Figur 4 gezeigt. Die Homologien finden sich insbesondere in konservierten Domänen, wie den Histidinboxen (Los & Murata, 1998. Biochem. Biophys. Acta 1394: 3-15; Shanklin, J et al. 1997. Proc. Natl. Acad. Sci. USA 92, 6743-6747). Darüberhinaus konnte wie bei anderen eukaryotischen delta-6-Desaturasen eine Cytochrom b5 Domäne (Lederer, F. (1994) Biochimie 76, 674-692; Cho et al. J. Biol. Chem. 1999, 274(1): 471-477) identifiziert werden. Obwohl die erfindungsgemäße Polypeptidsequenz sich als delta-6-Desaturase identifizieren läßt, unterscheidet sie sich wesentlich von anderen delta-6-Desaturasen. Auffällig ist vor allem, daß die Sequenz mit 352 Aminosäuren rund 20% kürzer als andere eukaryotischen delta-6-Desaturasen ist. Darüberhinaus weist die Sequenz in stark konservierten Bereichen eine Vielzahl von einzigartigen Abweichungen auf. So ist z.B. das bei anderen delta-6-Desaturasen zu 100% konservierte HHLFP-Motiv abgewandelt in HHFFP (siehe Figur 4). Die Identität der erfindungsgemäßen Polypeptidsequenz zu bekannten Desaturasen ist aus diesen Gründen überraschend gering.

Durch die gezielte Überexpression der Desaturase z. B. in Tetrahymena kann das Fettsäurespektrum signifikant modifiziert werden (s. Tabelle 1 und 2). Dabei kommt es zu einer Verschiebung des Verhältnisses der gesättigten und ungesättigten Fettsäuren hin zu deutlich mehr ungesättigten Fettsäuren. Von besonderem Interesse ist dabei die Steigerung der Produktivität der GLA, die so erreicht werden kann.

In einer bevorzugten Ausführungsform ist die erfindungsgemäße Nukleinsäure eine DNA oder RNA, vorzugsweise eine doppelsträngige DNA, und insbesondere eine DNA mit einer Nukleinsäuresequenz gemäß SEQ ID No 1 von Pos. 33 bis Pos. 1091. Die beiden Positionen bestimmen gemäß der vorliegenden Erfindung den Start und das Ende des kodierenden Bereiches.

Die Teile oder Fragmente der erfindungsgemäßen Nukleinsäuren können beispielsweise zur Herstellung einzelner Epitope, als Sonden zur Identifizierung funktioneller Varianten oder als Antisense-Nukleinsäuren verwendet werden. Beispielsweise eignet sich eine Nukleinsäure aus mindestens ca. 8 Nukleotiden als Antisense-Nukleinsäure, eine Nukleinsäure aus mindestens ca. 15 Nukleotiden als Primer beim PCR-Verfahren, eine Nukleinsäure aus mindestens ca. 20 Nukleotiden für die Identifizierung weiterer Varianten und eine Nukleinsäure aus mindestens ca. 100 Nukleotiden als Sonde.

In einer weiteren bevorzugten Ausführungsform enthält die erfindungsgemäße Nukleinsäure eine oder mehrere nicht-kodierende Sequenzen (UTR u.a.). Die nicht-kodierenden Sequenzen sind beispielsweise Intronsequenzen oder regulatorische Sequenzen, wie Promotor- oder Enhancer-Sequenzen, zur kontrollierten Expression des delta-6-Desaturase kodierenden Gens. Daher ist ein Gegenstand der Erfindung eine erfindungsgemäße Nukleinsäure gemäß SEQ ID No.: 3, welche aus *Tetrahymena thermophila* isoliert werden kann und die genomische Sequenz der delta-6-Desaturase mit Introns, Promoter und UTRs darstellt.

In einer weiteren Ausführungsform ist die erfindungsgemäße Nukleinsäure daher in einem Vektor, vorzugsweise in einem Expressionsvektor enthalten.

Die Expressionsvektoren können beispielsweise prokaryotische oder eukaryotische Expressionsvektoren sein. Beispiele für prokaryotische Expressionsvektoren sind für die Expression in E. coli z.B. der T7 Expressionsvektor pGM10 (Martin, 1996), welcher für einen N-terminalen Met-Ala-His6-Tag kodiert, der eine vorteilhafte Reinigung des exprimierten Proteins über eine Ni2+-NTA-Säule ermöglicht. Als eukaryotische Expressionsvektoren für die Expression in Saccharomyces cerevisiae eignen sich z.B. die Vektoren p426Met25 oder p426GAL1 (Mumberg et al. (1994) Nucl. Acids Res., 22, 5767), für die Expression in Insektenzellen z.B. Baculovirus-Vektoren wie in EP-B1-0127839 oder EP-B1-0549721 offenbart, und für die Expression in Säugerzellen z.B. SV40-Vektoren, welche allgemein erhältlich sind.

Im allgemeinen enthalten die Expressionsvektoren auch für die Wirtszelle geeignete regulatorische Sequenzen, wie z.B. den trp-Promotor für die Expression in E. coli (s. z.B. EP-B1-0154133), den ADH-2-Promotor für die Expression in Hefen (Russel et al. (1983), J. Biol. Chem. 258, 2674), den Baculovirus-Polyhedrin-Promotor für die Expression in Insektenzellen (s. z.B. EP-B1-0127839) oder den frühen SV40-Promotor oder LTR-Promotoren z.B. von MMTV (Mouse Mammary Tumour Virus; Lee et al. (1981) Nature, 214, 228).
Für die Transformation von und die Expression in Tetrahymena eignen sich z.B. die von Gaertig et al. ((1999) Nature Biotech. 17: 462-465) oder Gaertig & Kapler ((1999) Methods in Cell Biol. 62:485-500) beschriebenen Vektoren.

Die erfindungsgemäßen Nukleinsäuren können beispielsweise chemisch anhand der in SEQ ID No.: 1 und 3 offenbarten Sequenzen oder anhand der in SEQ ID No.: 2 offenbarten Peptidsequenz unter Heranziehen des genetischen Codes z.B. nach der Phosphotriester-Methode synthetisiert werden (s. z.B. Uhlman, E. & Peyman, A. (1990) Chemical Reviews, 90, 543, No. 4). Eine weitere Möglichkeit, die erfindungsgemäßen Nukleinsäuren in die Hand zu bekommen, ist die Isolierung aus einer geeigneten Genbank, von einem Organismus, der delta-6-Desaturase Aktivität besitzt, anhand einer geeigneten Sonde (s. z.B. Sambrook, J. et al. (1989) Molecular Cloning. A laboratory manual. 2nd Edition, Cold Spring Harbor, New York). Als Sonde eignen sich beispielsweise einzelsträngige DNA-Fragmente mit einer Länge von ca. 100 bis 1000 Nucleotiden, vorzugsweise mit einer Länge von ca. 200 bis 500 Nucleotiden, insbesondere mit einer Länge von ca. 300 bis 400 Nucleotiden, deren Sequenz aus der Nukleinsäuresequenz gemäß SEQ ID No.: 1 oder 3 abgeleitet werden kann.

Daher betrifft die Erfindung ebenfalls ein Verfahren zur Herstellung einer erfindungsgemäßen Nukleinsäure, wobei die Nukleinsäure chemisch synthetisiert oder anhand einer Sonde aus einer Genbank isoliert wird.

Ein weiterer Gegenstand der vorliegenden Erfindung ist auch das Polypeptid selbst mit einer Aminosäuresequenz gemäß SEQ ID No.: 2 und Teile davon mit mindestens 12 Aminosäuren, insbesondere mit mindestens 65 Aminosäuren und vor allem mit mindestens 150 Aminosäuren (nachfolgend "erfindungsgemäßes Polypeptid" genannt). Beispielsweise kann ein ca. 12, Aminosäuren-langes Polypeptid ein Epitop enthalten, das nach Kopplung an einen Träger zur Herstellung von spezifischen poly- oder monoklonalen Antikörper dient (siehe hierzu z. B. US 5,656,435). Polypeptide mit einer Länge von mindestens ca. 65 Aminosäuren können auch direkt ohne Träger zur Herstellung von poly- oder monoklonalen Antikörper dienen.

Des weiteren sind Polypeptide bevorzugt die konservierte Bereiche von Histidinboxen und eine Cytochrome b5 Domäne aufweisen. Besonders bevorzugt sind erfindungsgemäße Polypeptide die ein HHFFP - Motiv enthalten.

Ferner zählen hierzu auch Deletionen des Polypeptids im Bereich von ca. 1 - 60, vorzugsweise von ca. 1 - 30, insbesondere von ca. 1 - 15, vor allem von ca. 1 - 5 Aminosäuren. Beispielsweise kann die erste Aminosäure Methionin fehlen, ohne daß die Funktion des Polypeptids wesentlich verändert wird. Daneben zählen hierzu auch Fusionsproteine, die die oben beschriebenen erfindungsgemäßen Polypeptide enthalten, wobei die Fusionsproteine selbst bereits die Funktion einer delta-6-Desaturase haben oder erst nach Abspaltung des Fusionsanteils die spezifische Funktion bekommen können. Vor allem zählen hierzu Fusionsproteine mit einem Anteil von insbesondere nicht-ciliaten Sequenzen von ca. 1 - 200, vorzugsweise ca. 1 - 150, insbesondere ca. 1 - 100, vor allem ca. 1 - 50 Aminosäuren. Beispiele von nicht-Ciliaten Peptidsequenzen sind prokaryotische Peptidsequenzen, z.B. aus der Galactosidase von E. coli oder ein sogenannter Histidin-Tag, z.B. ein Met-Ala-His6-Tag. Ein Fusionsprotein mit einem sogenannten Histidin-Tag eignet sich besonders vorteilhaft zur Reinigung des exprimierten Proteins über Metallionen-haltige Säulen, beispielsweise über eine Ni2+-NTA-Säule. "NTA" steht für den Chelator "nitrilotriacetic acid" (Qiagen GmbH, Hilden).

Die Teile des erfindungsgemäßen Polypeptids repräsentieren beispielsweise Epitope, die spezifisch von Antikörpern erkannt werden können.

Das erfindungsgemäße Polypeptid kann beispielsweise durch Expression der erfindungsgemäßen Nukleinsäure in einem geeigneten Expressionssystem, wie oben bereits beschrieben, nach dem Fachmann allgemein bekannten Methoden hergestellt werden. Als Wirtszellen eignen sich beispielsweise die E. coli Stämme DH5, HB101 oder BL21, der Hefestamm Saccharomyces cerevisiae, die Insektenzelllinie Lepidopteran, z.B. von Spodoptera frugiperda, oder tierische Zellen wie COS, Vero, 293 und HeLa, die alle allgemein erhältlich sind.

Insbesondere die genannten Teile des Polypeptids können auch mit Hilfe der klassischen Peptidsynthese (Merrifield-Technik) synthetisiert werden. Sie eignen sich insbesondere zur Gewinnung von Antiseren, mit deren Hilfe geeignete Genexpressionsbanken durchsucht werden können, um so zu weiteren funktionellen Varianten des erfindungsgemäßen Polypeptids zu gelangen.

Ein weiterer Gegenstand der vorliegenden Erfindung bezieht sich daher auf ein Verfahren zur Herstellung eines erfindungsgemäßen Polypeptids, wobei eine erfindungsgemäße Nukleinsäure in einer geeigneten Wirtszelle exprimiert und gegebenenfalls isoliert wird.

Ein anderer Gegenstand der vorliegenden Erfindung bezieht sich auch auf Antikörper, die mit dem erfindungsgemäßen Polypeptid spezifisch reagieren, wobei die oben genannten Teile des Polypeptids entweder selbst immunogen sind oder durch Koppelung an geeignete Träger, wie z.B. bovines Serumalbumin, immunogen gemacht bzw. in ihrer Immunogenität gesteigert werden können.

Die Antikörper sind entweder polyklonal oder monoklonal. Die Herstellung, die ebenfalls einen Gegenstand der vorliegenden Erfindung darstellt, erfolgt beispielsweise nach allgemein bekannten Methoden durch Immunisieren eines Säugetiers, beispielsweise eines Kaninchens, mit dem erfindungsgemäßen Polypeptid oder den genannten Teilen davon, gegebenenfalls in Anwesenheit von z.B. Freund's Adjuvant und/oder Aluminiumhydroxidgelen (s. z.B. Diamond, B.A. et al. (1981) The New England Journal of Medicine, 1344). Die im Tier aufgrund einer immunologischen Reaktion entstandenen polyklonalen Antikörper lassen sich anschließend nach allgemein bekannten Methoden leicht aus dem Blut isolieren und z.B. über Säulenchromatographie reinigen. Bevorzugt wird eine Affinitätsreinigung der Antikörper, bei der beispielsweise das C-terminale Desaturase-Fragment an eine NHS-aktivierte HiTrap-Säule gekoppelt wurde.

Monoklonale Antikörper können beispielsweise nach der bekannten Methode von Winter & Milstein (Winter, G. & Milstein, C. (1991) Nature, 349, 293) hergestellt werden.

Obwohl bereits delta-6-Desaturasen aus anderen Organismen beschrieben wurden, bietet sich Tetrahymena, aufgrund der besonders hohen Raum/Zeit-Ausbeute bei der Produktion von GLA sowohl als Ausgangspunkt für die Erzeugung hochproduktiver, kommerziell bedeutender Stämme durch gentechnische Methoden, als auch als Quelle für die Gene der PUFA-Biosynthese an. In der vorliegenden Erfindung wird dementsprechend die delta-6-Desaturase und ihre Verwendung aus dem GLA-produzierenden Ciliaten *Tetrahymena thermophila* beschrieben.

Die gezielte Modifizierung der Zusammensetzung des Fettsäurespektrums durch gentechnische Methoden wird in Napier J et al. (Curr. Opin. Plant Biol. (1999) 123-127), Murphy & Piffanelli (Soc. Exp. Biol. Semin. Ser. 67 (Plant Lipid Biosynthesis), (1998) 95-130) und Facciotti & Knauf (In: Adv. Photosynth. 6: Lipids in Photosynthesis: Structure, Function and Genetics. Siegenthaler & Murata (eds.) Kluwer Academic Publishers. Netherlands. (1998) 225-248) beschrieben.

In den Schriften WO98/46763, WO98/4676 und WO98/4676 werden Desaturasen aus dem Pilz Mortierella und die Verwendung der Gene zur Produktion von PUFAs, sowie einige teilweise partielle Sequenzen von Desaturasen beschrieben. In WO93/06712 und WO96/21022 werden Δ6-Desaturasen aus einem Cyanobakterium und aus Borretsch sowie die Verwendung dieser Sequenzen zur Produktion von PUFAs in Cyanobakterien und Pflanzen beschrieben. In WO99/2711 wird eine Desaturase aus Nematoden und die Verwendung zur Produktion von PUFAs beschrieben. Aus der Literatur sind ebenfalls delta-6-Desaturasen bekannt. Es handelt sich dabei jedoch vor allem um delta-6-Desaturasen aus Pflanzen (Borretsch (Sayanova et al. (1997) Proc. Natl. Acad. Sci. USA 1997, 94: 4211-4216), Phycsomitrella (Girke et al. (1998) Plant-J. 1998 15(1): 39-48), Sonnenblume (Sperling et al. (1995) Eur. J. Biochem. 1995, 232: 798-805)), Pilzen (Mortierella), Tieren (Maus, Ratte, Caenorhabditis (Napier et al. (1998) Biochem. J. 330(2), 611-614)), Cyanobacterium (Reddy et al. (1993) Plant Mol. Biol. 1993, 293-300).

Ziel ist im allgemeinen die funktionelle, heterologe Expression dieser Gene in Kulturpflanzen, insbesondere Ölsaaten wie z.B. Raps, Sonnenblume und anderen. Die GLA-Ausbeuten sind in allen bisher veröffentlichten Fällen jedoch entweder sehr gering und/oder die durch "genetic engineering" erzeugten GLA-produzierenden Organismen mit delta-6-Desaturase-Aktivität sind ohne kommerzielle Bedeutung (Knutzon & Knauf (1998) Soc. Exp. Biol. Semin. Ser. 67:287-304).
Die Probleme und Schwierigkeiten das Fettsäurespektrum in transgenen Pflanzen gezielt zu modifizieren werden z.B. von Murphy (Current Opinion in Biotechnology (1999) 10:175-180) und Knutzon & Knauf (1998) beschrieben.

Aufgrund des beschriebenen hohen GLA-Gehalts von Tetrahymena ist es vorteilhaft die Gene der PUFA-Biosynthese aus diesem Organismus für die Entwicklung hochproduktiver, kommerziell bedeutender Stämme durch gentechnische Methoden zu benutzen. Durch die Möglichkeit, Tetrahymena gut in Massenkultur mit einer hohen Zelldichte zu kultivieren, ist es darüber hinaus vorteilhaft Tetrahymena selbst zur Erzeugung solcher hochproduktiver, kommerziell interessanter Stämme durch gentechnische Methoden zu benutzen. Desweiteren können neben Tetrahymena auch andere Organismen mit Hilfe der erfindungsgemäßen Nukleinsäure zur Produktion von GLA oder anderen delta 6-ungesättigten Fettsäuren benutzt werden.

Die Herstellung von GLA durch Fermentation von Tetrahymena ist besonders vorteilhaft aufgrund des recht einfachen Fettsäurespektrums verglichen mit höheren Organismen. Darüber hinaus wird eine fermentative Produktion nicht von externen Faktoren wie Wetter, Nahrungsangebot etc. beinflusst. Ausserdem ist ein auf diese Weise gewonnenes Produkt weitgehend frei von Verunreinigungen, wie sie z.B. bei aus der Natur gewonnenen Produkten durch Umweltverschmutzung hervorgerufen werden können.

Mit Hilfe der erfindungsgemäßen Nukleinsäuren, kodierend für eine ciliatenspezifische delta-6-Desaturase aus Tetrahymena, können transgene Organismen erzeugt werden, welche GLA und delta 6-ungesättigte Fettsäuren produzieren, bzw. deren Gehalt an solchen Fettsäuren gegenüber Wildtypzellen (hier: *Tetrahymena thermophila)* deutlich erhöht ist (siehe Tabelle 1 und 2). Dabei handelt es sich bevorzugt um Ciliaten, besonders bevorzugt Tetrahymena, welche die erfindungsgemäße Nukleinsäuresequenz enthalten, bzw. in funktioneller Weise exprimieren. Die Expression der Desaturase in dieser Weise führt zu einem relativen Anstieg von delta-6-ungesättigten Fettsäuren, bzw. davon abgeleiteten Folgeprodukten, basierend auf einer veränderten Konzentration von Enzymen und Substraten der PUFA-Synthese. Die Erfindung findet Anwendung in der kommerziellen Produktion von PUFAs, insbesondere von GLA und davon abgeleiteten PUFAs oder anderen Folgeprodukten, die sich von GLA ableiten lassen, bzw. Δ6-ungesättigten Fettsäuren (s. Figur 1: PUFA-Biosynthese). Neben GLA kann so z.B. durch die Desaturierung der ALA auch Stearidonsäure (18:4 Δ6,9,12,15) hergestellt werden, ein industriell vielfach genutzter Rohstoff.

In einer besonderen Ausführungsform kann durch die Verwendung der delta-6-Desaturase kodierenden erfindungsgemäßen Nukleinsäuren in funktioneller Kombination mit geeigneten regulativen Sequenzen eine verstärkte Expression des Enzyms der GLA-Gehalt in GLA-produzierenden Organismen erhöht werden, bzw. GLA in LAproduzierenden Organismen produziert werden. Von besonderem Interesse sind dabei z.B. ölproduzierende Organismen wie Sonnenblume, Raps, Soja aber auch andere. Darüber hinaus können durch die gleichzeitige Verwendung z. B. einer delta 12-Desaturase (z. B. Sakuradani E et al. (1999) Eur. J. Biochem. 261:812-820, Okuley et al. Plant Cell (1994) 6(1) 147-58) die besagten PUFAs in Organismen oder Zellen produziert werden, die keine oder nur wenig LA enthalten. Ebenfalls eine Kombination der drei an der GLA-Bildung beteiligten Desaturasen Δ6, Δ9 und Δ12 zur Produktion von GLA und delta 6-ungesättigte Fettsäuren ist möglich. Des weiteren ist die Kombination mit weiteren Genen der PUFA-Biosynthese (vgl. Figur 1) eine weitere bevorzugte Ausführung der Erfindung, wobei die GLA und delta 6-ungesättigte Fettsäuren mittels weiterer Enzyme umgesetzt werden, für die GLA als Substrat dient, wodurch z.B. ARA (20:4) und andere Moleküle, die sich von GLA ableiten, hergestellt werden können.

Außerdem kann die Erfindung zur Herstellung neuer GLA-haltiger Nahrungsquellen, bzw. von Nahrungsquellen die reich an Molekülen (insbesondere PUFAs, z. B. ARA) sind, die sich von GLA oder anderen Δ6-ungesättigten Fettsäuren ableiten lassen, benutzt werden.

Die vorliegende Erfindung beschreibt weiterhin Expressionskonstrukte, die das delta-6-Desaturase Gen oder Teile davon enthalten, sowie die funktionelle Kombination der delta-6-Desaturase kodierenden Sequenz mit heterologen regulativen Sequenzen.

Ein weiterer Gegenstand der Erfindung ist die Herstellung transgener Organismen mit erhöhtem GLA-Gehalt (siehe Tabellen 1 und 2), durch die Verwendung der beschriebenen delta-6-Desaturase kodierenden DNA-Sequenz und der beschriebenen funktionellen Konstrukte des delta-6-Desaturase Gens.

Für die Isolierung von genomischer DNA und von mRNA, sowie die Herstellung von genomischer und cDNA-Banken sind eine Vielzahl gut etablierter Methoden bekannt (z.B. in Sambrook et al. (1989) in Molecular Clonning: A Laboratory Manual, Cold Spring Harbor, NY). Die Herstellung geeigneter Vektoren, welche die in der Erfindung beschriebene Desaturase, bzw. Teile daraus enthalten, können mit dem Fachmann bekannten Methoden, wie sie z. B. in Ausubel et al. (Ausubel et al. (1995), Current Protocols in Molecular Bioloqy, Green Publishing Associates, New York) und Sambrook et al. (1989) beschrieben sind, hergestellt werden.

Vektoren, welche die delta-6-Desaturase kodierende Sequenz enthalten, können durch Infektion, Transfektion, Elektroporation, Partikelbeschuß und anderen Methoden in Zellen eingeschleust werden. Mit Transformation ist hier generell die Einbringung fremder DNA in eine Zelle gemeint. Die Methoden dafür sind gut etabliert und können in für den Fachmann bekannter Weise durchgeführt werden (z. B. Sambrook et al. (1989), Potrykus I (1991) Annu. Rev. Plant Biol. Plant Mol. Biol. 42:205-225, Christou P (1993) Curr. Opp. Biotech. 4:135-141).

Ebenso werden Vektoren beschrieben, welche die DNA-Sequenz der vorliegenden Erfindung oder Teile der Sequenz in funktioneller Kombination mit Promotoren, oder anderen regulativen Elementen, welche in einer Wirtszelle aktiv sind, enthalten. In einer bevorzugten Ausführung handelt es sich bei diesen regulativen Elementen um Nukleinsäuresequenzen, welche in Ciliaten, insbesondere Tetrahymena, funktionell aktiv sind, wie z. B die Promoteren für Histon H4, α- und β-Tubulin und andere.

Ferner werden in der vorliegenden Erfindung Organismen beschrieben, welche die beschriebene Delta-6-Desaturase kodierende Sequenz rekombinant exprimieren. Dadurch besteht neben der Möglichkeit der Produktion von Δ6-PUFAs in diesen Organismen z. B. auch die Möglichkeit die rekombinante delta-6-Desaturase oder Teile davon durch Standardmethoden der Proteinreinigung (z. B. Ausube! et al. (1995)) zu isolieren. Vektoren für eine Expression der delta-6-Desaturase kodierenden Sequenz in verschiedenen Organismen können nach dem Fachmann bekannter Weise hergestellt werden. Detaillierte Informationen über geeignete Vektoren finden sich z.B. in Sambrook et al. (1989), Goeddel, ed. (1990) Methods in Enzymology 185 Academic Press, und Perbal (1988) A Practical Guide to Molecular Cloning, John Wiley and Sons, Inc.. Solche Vektoren enthalten vorzugsweise Sequenzelemente, welche die Expression beeinflussen, wie Promotoren, Enhancer-Elemente, "upstream" aktivierende Sequenzen etc.. Für die Expression eignen sich sowohl induzierbare als auch konstitutive Promotoren, oder z. B. gewebespezifische Promotoren. Für die Expression in pflanzlichen Zellen eignet sich z. B. der "cauliflower mosaic virus" (CaMV) 35S Promoter (Restrepo et al. (1990) Plant Cell 2 987) oder z. B. Promotoren, die bei der Samenentwicklung aktiviert werden.

Vorzugsweise handelt es sich bei den benutzten Vektoren um Shuttle-Vektoren (Wolk et al. (1984) Proc. Natl. Acad. Sci. USA, 1561-1565, Bustos et al. (1991) J. Bacteriol. 174: 7525-7533).

In einer bevorzugten Ausführungsform wird die erfindungsgemäße Nukleinsäure unter Kontrolle eines starken Promotors (wie z. B dem Tetrahymena β-Tubulin-Promoter, Gaertig et al. (1999) Nature Biotech.) in Tetrahymena exprimiert. Die Transformation kann vorzugsweise nach Gaertig et al. (1999) Nature Biotech. 17: 462-465 (oder z. B. nach Gaertig & Gorovsky (1992) Proc. Natl. Acad. Sci. USA 89:9196-9200) beschriebenen Methoden erfolgen. Als regulative Elemente für die Expression können z. B. die Promotoren von α- oder β-Tubulin aus *Tetrahymena thermophila* benutzt werden. Die transformierten Tetrahymena werden in selektiven Medien identifiziert, angereichert und kultiviert. Aus den Zellen können die Lip(o)ide nach Standardmethoden isoliert werden (z.B. Dahmer et al., (1989) Journal of American Oil Chemical Society 66, 543). Die Methylesther der Fettsäuren können durch Gaschromatographie analysiert werden.

Die in der vorliegenden Erfindung beschriebenen isolierten Nukleinsäuren oder Teile daraus können auch zur Isolierung verwandter Gene aus anderen Organismen, insbesondere z. B. aus anderen Protisten, insbesondere Ciliaten, benutzt werden. Für die Isolierung homologer Gene kann die erfindungsgemäße Nukleinsäure oder Teile davon als markierte Probe eingesetzt werden. Durch die Hybridisierung der Probe an isolierte Nukleinsäuren aus anderen Organismen können homologe Nukleinsäure-Sequenzen identifiziert und isoliert werden. Die Markierung der Probe kann in einer für den Fachmann bekannten Weise durchgeführt werden (Ausubel, Sambrook (supra)). Für die Markierung der Probe eignen sich z.B. radioaktive Nukleotide oder Nukleotide die mit detektierbaren Molekülen wie z.B. fluoresziernden Molekülen, Digoxigenin, Biotin, magnetischen Molekülen oder Enzymen verknüpft sind. Die Identifizierung und Isolierung homologer DNA-Sequenzen erfolgt durch den Nachweis der Markierung nach einer Hybridisierung der Probe an heterologe DNA. Für die Suche nach homologen Sequenzen bieten sich cDNA-Banken oder genomische Banken an. Für den Nachweis homologer Sequenzen eigenen sich darüber hinaus Southern und Northern-Blots. Alternativ kann homologe DNA, die mit der markierten Probe hybridisiert, auch durch selektives Zurückhalten der markierten Probe (z.B. mit Hilfe eines Magneten) isoliert werden.

Die Isolierung und Klonierung homologer Gene durch Kreuzhybridisierung kann durch dem Fachmann bekannte Methoden, wie sie z. B. in Ausubel et al. (1995, Current Protocols in Molecular Biology, Green Publishing Associates, New York) oder Sambrook et al. (1989, Molecular Cloning) beschrieben sind, erfolgen.

Auf der Basis der isolierten DNA-Sequenz und der dadurch kodierten Proteinsequenz können zudem Oligonukleotide entworfen werden, mit deren Hilfe homologe Nukleinsäuresequenzen mittels Polymerasekettenreaktion (PCR) amplifiziert werden können.

Eine weitere Möglichkeit zur Isolierung homologer Proteine besteht in der Detektion mit spezifischen Antikörpern gegen das durch die Sequenz der vorliegenden Erfindung kodierten Protein oder Teile davon (z. B. Peptid-Antikörper).

### Beschreibung der wichtigsten Sequenzen und Figuren:

- **SEQ ID No: 1:**: Nukleotidsequenz der delta-6-Desaturase kodierenden cDNA- der delta-6-Desaturase von *Tetrahymena thermophila.* Start- und Stop- Codon sind hervorgehoben.
- **SEQ ID No: 2:**: aus der SEQ ID No.: 1 abgeleitete Proteinsequenz der Tetrahymena delta-6-Desaturase, unter Berücksichtigung des speziellen Ciliaten- Codon-Gebrauchs (Wuitschick JD, Karrer KM (1999), oder CUTG (Codon usage tabulated from Genbank): http://www.dna.affrc.go.jp/~nakamura/CUTG.html).
- **SEQ ID No: 3:**: genomische Nukleotidsequenz der delta-6-Desaturase von *Tetrahymena thermophila.*

- **Figur 1**:: Schematische Darstellung der PUFA-Biosynthese.
- **Figur 2**:: Ergebnis eines BLASTP - Datenbank - Vergleichs der Proteinsequenz gemäß SEQ ID No.: 2 mit Proteindatenbanken.
- **Figur 3:**: Alignment der Proteinsequenz gemäß SEQ ID No.: 2 mit bekannten Desaturasen.
- **Figur 4:**: Multiples Alignment der Polypeptidsequenz gemäß SEQ ID No.: 2 aus tetrahymena mit bekannten Desaturasen. Die Histidin-Boxen sowie das konservierte HPGG-Motiv aus der Cytochrom b5 Domäne sind unterstrichen.
- **Figur 5**:: Schematische Darstellung der Genstruktur der delta-6-Desaturase aus Tetrahymena gemäß SEQ ID No.: 1 und 3.
- **Figur 6**:: Herstellung des pBDES6 delta-6-Desaturase Expressionskonstruktes.

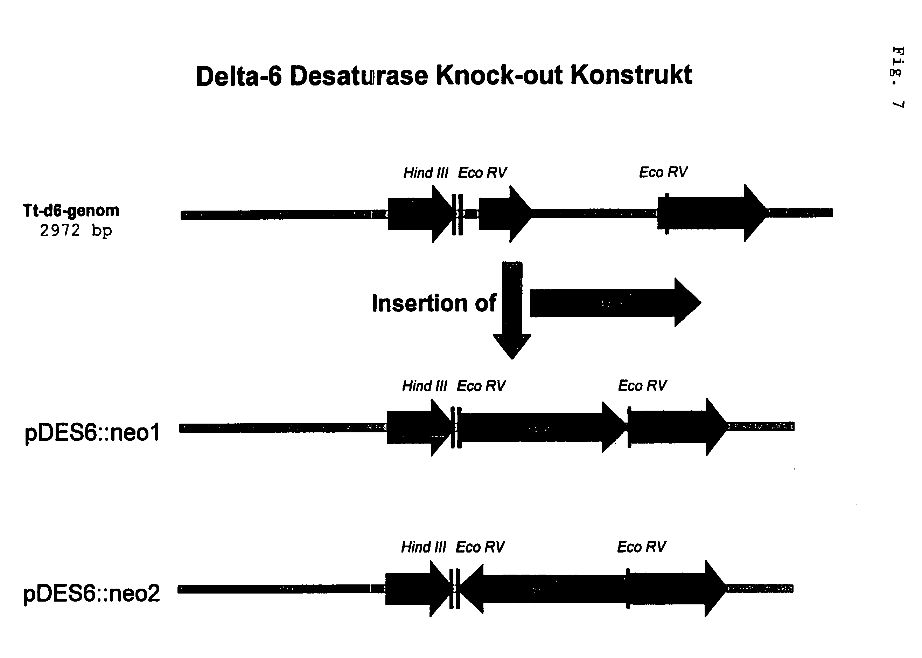

- **Figur 7**:: Herstellung der pgDES6::neo Knockoutkonstrukte.
- **Figur 8**:: Vergleich des Fettsäurespektrums (Hauptfettsäuren) der Tetrahymena pBDES6-Transformanten (AX601 und AX604) im Vergleich zum Tetrahymena-Wildstamm (CU522)

### BEISPIELE

Die nachfolgenden Beispiele dienen zur Erläuterung der Erfindung, ohne die Erfindung auf diese Beispiele einzugrenzen.

### BEISPIEL 1:

### Organismen und Kultivierungsbedingungen

*Tetrahymena thermophila* (Stämme B1868 VII, B2086 II, B*VI, CU427, CU428, CU522, zur Verfügung gestellt von Dr. J. Gaertig, University of Georgia, Athens, GA, USA) wurden in modifiziertem SPP-Medium (2% Proteosepeptone, 0,1% Hefeextrakt, 0,2% Glucose, 0,003% Fe-EDTA (Gaertig et al. (1994) PNAS 91:4549-4553)) bzw. Magermilchmedium (2% Magermilchpulver, 0,5% Hefeextrakt, 1 % Glucose, 0,003% Fe-EDTA) oder MYG-Medium (2% Magermilchpulver, 0,1 % Hefeextrakt, 0,2% Glucose, 0,003% Fe-EDTA) mit Zusatz von Antibiotika-Lösung (100 U/ml Penicillin, 100 µg/ml Streptomycin und 0,25 µg/ml Amphotericin B (SPPA-Medium)) bei 30 °C in 50 ml Volumen in 250 ml Erlenmeyerkolben unter Schütteln (150 U/min) kultiviert.

Plasmide und Phagen wurden in *E. coli* XL1-Blue MRF', TOP10F' oder JM109 vermehrt und selektiert. Die Kultivierung der Bakterien erfolgte unter Standardbedingungen in LB- oder NZY-Medium, mit Antibiotika in Standardkonzentrationen (Sambrook et al. (1989) Molecular Cloning: A Laboratory Manual, Cold Spring Harbor Laboratory, Cold Spring, New York).

### BEISPIEL 2:

### Herstellung einer Tetrahymena thermophila cDNA-Bibliothek

Gesamt RNA aus *Tetrahymena thermophila* wurde nach der Guanidinthiocyanat/ Phenol/Chloroform Methode isoliert (Chomzynski & Sacchi (1987) Anal. Biochem. 161: 156-159). Aus der Gesamt-RNA wurde die mRNA mit Hilfe von Oligotex-dT-Kügelchen (Qiagen) isoliert. Die Synthese der cDNA erfolgte nach dem Stratagene ZAP Express cDNA Synthesis and Cloning Kit. Nach Ligation der EcoR I-Adapter und Verdau mit Xho I wurde die DNA über ein Agarosegel aufgetrennt und größenfraktioniert (S: 500 - 1500 bp, B: größer 1500 bp). Die DNA wurde aus dem Gel isoliert (Qiaquick Gel Extraktion Kit, QIAGEN) und in den Eco RI und Xho I geschnittenen ZAP Express Vektor ligiert. Die ligierte DNA wurde in-vitro in Phagen verpackt (Stratagene Gigapack III Gold) und die Phagen wurden in E. coli XL1-Blue MRF' vermehrt. Die S-cDNA-Bank enthielt ca. 5 x 10⁵ Klone mit einer durchschnittlichen Insertgröße von 1,1 kb, die B-cDNA-Bank enthielt ca. 6 x 10⁴ Klone mit einer durchschnittlichen Insertgröße von 2 kb.

### BEISPIEL 3:

### RT-PCR mit delta-6-Desaturase spezifischen Primern

Durch Sequenzvergleiche bekannter Desaturasen konnten konservierte Bereiche identifiziert werden. Für die besonders stark konservierten Aminosäurebereiche WWKWNHNAHH (SEQ ID No.: 4) und GGLQFQIEHHLFP (SEQ ID No.: 5)
wurden unter Berücksichtigung des besonderen Ciliaten-Codon- bzw. Tetrahymena-Codon-Gebrauchs PCR-Primer entworfen (Wuitschick JD, Karrer KM (1999) Analysis of genomic G + C content, codon usage, initiator codon context and translation termination sites in Tetrahymena thermophila. J. Eukaryot. Microbiol. 46(3):239-47; Martindale (1989) J. Protozool. 36,1: 29-34, CUTG, (Codon Usage Tabulated from Genbank): http://www.dna.affrc.go.jp/~nakamura/CUTG.html). Primer 1 (sense): 5'-TGGTGGAARTGGAMNCAYAA-3', (SEQ ID No.: 6) Primer 2 (antisense): 5'-CGDGGRAANARRTGRTGTTC-3' (SEQ ID No.: 7).
100 ng isolierte mRNA wurde für die Erststrangsynthese mit AMV-Reverser Transkriptase (Boehringer Mannheim) eingesetzt. Die Reaktion erfolgte nach dem Hersteller-Protokoll in 20 µl Volumen: 50 mM Tris-HCl (pH 8,5), 8 mM MgCl₂, 30 mM KCI, 1 mM DTT, 1 mM dNTPs, 2 pmol Oligo-dT-Ankerprimer (5'- GACCACGCGTATCGATGTCGACT(16)V-3'; SEQ ID NO.: 8), 2 units AMV-Reverse Transkriptase, 60 min. bei 55 °C, anschließend 10 min 65 °C. 1/10 dieser Erststrangreaktion wurde für die PCR eingesetzt. Die PCR erfolgte in 25 µl Volumen mit: 1 x Qiagen HotStarTaq PCR-Puffer (QIAGEN), pH 8,7 (20 °C), je 10 pmol der delta-6-Desaturase spezifischen Primer, je 200 µM dNTPs, 1,5 mM MgCl₂, 1 unit HotStarTaq-Polymerase (Qiagen). Die PCR wurde unter folgenden Bedingungen durchgeführt: Anfangsdenaturierung bei 95 °C für 15 min, anschließend folgten 35 Zyklen mit jeweils 94 °C für 30 sec, 45 °C für 30 sec und 72 °C für 1 min. Abschließend 10 min 72 °C. Die PCR-Fragmente wurden durch T/A Klonierung (Invitrogen) in den Vektor pCR 2.1 ligiert und in E. coli TOP10F' (Invitrogen) vermehrt. Von positiven Klonen wurde Plasmid-DNA isoliert (Qiaprep Spin, QIAGEN) und sequenziert.

### BEISPIEL 4:

### Isolierung der vollständigen delta-6-Desaturase kodierenden cDNA

Auf der Basis der so ermittelten Sequenz wurden neue Oligonukleotide für die PCR entworfen:
Primer d6/1-F (sense): 5'- GGAATCACAATCAACATCATATGTTCAC -3' (SEQ ID No.: 9) und
Primer d6/1-R (antisense): 5'- CTTCGTCCTTTAGAATGTTGTTTGTGAAC -3' (SEQ ID No.: 10).

Die Isolierung der vollständigen delta-6-Desaturase kodierenden cDNA erfolgte durch PCR mit diesen Primern in Kombination mit Vektorspezifischen Primern (T3 und T7) aus der cDNA-Bank. Von der cDNA-Bank wurden 2 µl (10⁵ pfu/µl) für eine PCR (s.o) eingesetzt. Die PCR erfolgte abweichend von den oben angegebenen Bedingungen nach folgendem Protokoll: 15 min 95 °C Denaturierung, anschließend 35 Zyklen mit 20 sec. 94 °C, 20 sec 57 °C, 1 min. 72 °C. Abschließend 10 min 72 °C. Die PCR-Produkte wurden mit den auch für die PCR eingesetzten Primern sequenziert. Auf der Basis der so gewonnenen Sequenzinformationen wurde ein neuer Primer entworfen, der am 5'-Ende der cDNA-Sequenz lag:
Primer d6-5'-F: AGTAAGCAAACTAAATTTAAAAAACAAGC (SEQ ID NO.: 11)

Mit Hilfe dieses Primers in Verbindung mit einem vektorspezifischen Primer konnte die vollständige cDNA-Sequenz durch PCR (PCR-Bedingungen s.o.) amplifiziert und isoliert werden. Durch die Klonierung dieses PCR-Produktes in den Vektor pCR 2.1 erhielt man das Plasmid pDES6.

### BEISPIEL 5:

### Herstellung einer Tetrahymena thermophila genomischen DNA-Bibliothek

Genomische DNA wurde mit Hilfe der Harnstoff-Methode (Gaertig et al. 1994) aus Tetrahymena isoliert und mit Eco R I geschnitten. Die geschnittene DNA wurde in einen ebenfalls Eco R I geschnittenen Lambda-Vektor (Zap Express, Stratagene) ligiert. Die weitere Durchführung entsprach der Vorgehensweise bei der cDNA-Bibliothek.

### BEISPIEL 6:

### Isolierung der genomischen Sequenz der delta-6-Desaturase

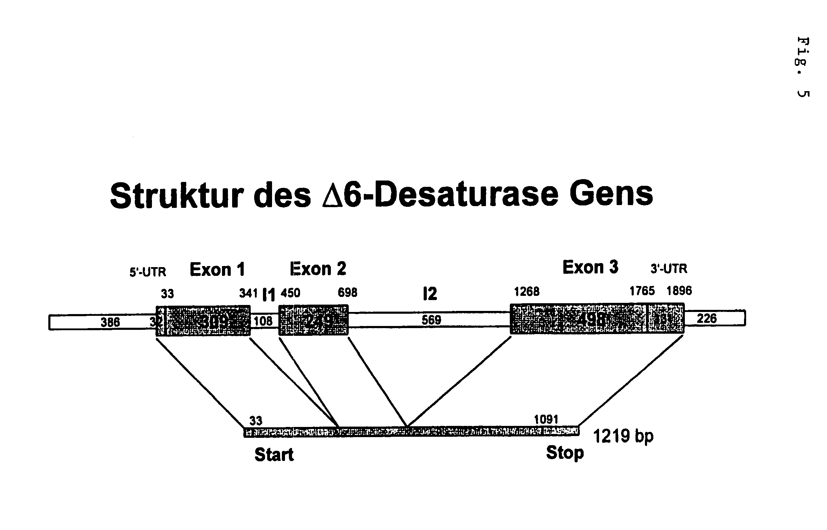

Die genomische Sequenz der delta-6-Desaturase wurde mit Hilfe der PCR ermittelt. Zum einen wurde mit Primern vom 5'- und 3'-Ende der cDNA:
d6-5'-F: AGTAAGCAAACTAAATTTAAAAAACAAGC (SEQ ID No.: 12) d6-3'-R: GGTCCTTCATGAATCTTAAGGTTCCACTTC (SEQ ID No.: 13) aus genomischer DNA ein ca. 2200 bp großes PCR-Produkt erzeugt, welches die gesamte kodierende Sequenz und Introns enthielt. Um flankierende Sequenzen des delta-6-Desaturase Gens zu isolieren wurde das Genome Walker System (Clonetech) benutzt. Mit Hilfe der universellen Primer aus diesem System und spezifischen Primern auf der Basis der ermittelten delta-6-Desaturase Sequenz
d6-5'-R: CTTAAGTCTTATCAACTCCCATAATGC (SEQ ID No.: 14)
d6-3'-F: GAAGTGGAACCTTAAGATTCATGAAGGACC (SEQ ID No.: 15) konnten flankierende Bereiche des delta-6-Desaturase Gens aus Tetrahymena isoliert werden. Die vollständige Struktur der genomischen Sequenz ist in Figur 5 dargestellt.

### BEISPIEL 7:

### Herstellung der Expressionskonstrukte pBDES6

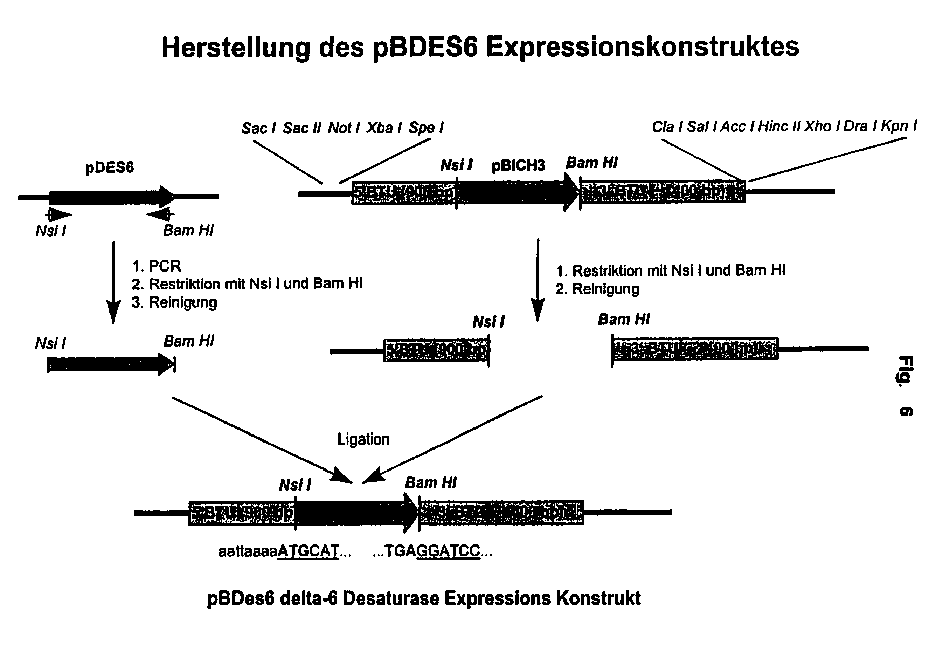

Der Vektor pBICH3 (Gaertig et al. 1999 Nature Biotech. 17: 462-465) enthält die kodierende Sequenz des *Ichthyophthirius* I-Antigen (G1) Präprotein flankiert von den nicht kodierenden, regulativen Sequenzen des *Tetrahymena thermophila* BTU1-Gens. Ein modifiziertes Plasmid (pBICH3-Nsi) mit einer Nsi I Schnittstelle am Start (zur Verfügung gestellt von J. Gaertig, University of Georgia, Athens, GA, USA) wurde benutzt, um das delta-6-Desaturase Expressionskonstrukt pBDES6 herzustellen. Dazu wurden mittels PCR Nsi I und Bam HI Schnittstellen am Start und Stop der kodierenden Sequenzen der delta-6-Desaturase von *Tetrahymena* eingefügt. Für die PCR wurden isolierte Plasmide, welche die vollständigen cDNA-Sequenzen der delta-6-Desaturase enthalten (pDES6) als Template eingesetzt. Die Primer
D6-Nsi-F: 5'-GCATTATGCATGTTGATAAGACTTAAGAAG-3' (SEQ ID No.: 16)
D6-Bam-R: 5'-TATGGATCCTCAAAGGTGAGATTTTTCAAAAATAG-3' (SEQ ID No.: 17)
erzeugten PCR-Produkte, welche die vollständige kodierende Sequenz der delta-6-Desaturase, flankiert von Nsi I und Bam HI Schnittstellen, enthielten. Die PCR-Produkte und das Plasmid pBICH3-Nsi wurden mit den Restriktionsenzymen Nsi I und Bam HI geschnitten, über ein Agarosegel gereinigt und ligiert (s. Abb. Plasmidkonstruktion). Die so entstandenen Expressionskonstrukte pBDES6 enthielten die vollständige delta-6-Desaturase kodierende Sequenz inseriert in einem korrekten Leseraster in die regulatorischen Sequenzen des BTU1 Gens (siehe Figur 6). Für die Transformation von Tetrahymena wurden die Konstrukte durch einen Verdau mit den Restriktionsenzymen Xba I und Sal I linearisiert.
Bei einer erfolgreichen Transformation wurde durch homologe Rekombination das BTU1-Gen durch diese Konstrukte ersetzt, wodurch eine Resistenz der Zellen gegenüber Paclitaxel vermittelt wurde.

### BEISPIEL 8:

### Bestimmung des Fettsäurespektrums der Transformanten

Die Bestimmung des Fettsäurespektrums erfolgte mittels Gaschromatographen (HP GC 6890) mit Flammenionisationsdetektor (Hewlett-Packard Company, Wilmington, USA). Als Säule diente eine FFAP (Free Fatty Acid Phase) Permbond (Macherey & Nagel GmbH, Düren). Die Identifizierung der Fettsäuren erfolgte durch den Vergleich mit Retentionszeiten von Fettsäuremethylester-Standards. Auf der Basis der bekannten Konzentration des Standards konnte die Konzentration der Fettsäuren in den Proben bestimmt werden.
Zur Bestimmung des Fettsäurespektrums wurden die isolierten Transformanten in MYG-Medium bei 30 °C, 150 RPM für 24-96 h kultiviert. 50 ml der Kultur wurden bei 1500 g für 15 min zentrifugiert, der Überstand verworfen, das Pellet bei -80 °C eingefroren und anschliessend gefiergetrocknet. 50 mg der lyophillisierten Probe wurden eingewogen und mit 1 ml 20%iger methanolischer HCl und 1 ml methanolischer Standard-Lösung (1 mg/ml) versetzt. Zur Freisetzung der Fettsäuren und deren Umesterung in Fettsäuremethylester wurden die Proben im geschlossenen Reagenzglas zwei Stunden bei 60°C im Wasserbad gerührt und auf Raumtemperatur abgekühlt. Zum Neutralisieren der Probe wurde anschließend 1 ml wässrige, gesättigte NatriumhydrogencarbonatLösung zugegeben und vorsichtig gemischt. Durch Zugabe von n-Hexan wurden die Fettsäuremethylester extrahiert. Anschließende wurde der Ansatz kräftig durchmischt und durch Zentrifugation für 2 min. bei 4300 rpm eine Phasentrennung erreicht. Etwa 2/3 der oberen, organischen Phase wurden entnommen und 1 µl der Probe wurde auf die GC-Säule gespritz und analysiert.

**Tabelle 1: GLA-Gehalt der Tetrahymena pBDES6-Transformanten (AX601 und AX604) im Vergleich zumTetrahymena-Wildstamm (CU522) nach 50 h Kultivierung. Angegeben ist der prozentuale Anteil der GLA am Gesamtfettsäurespektrum sowie die prozentuale Differenz der Transformanten im Vergleich zum nicht-transformierten Tetrahymena Stamm CU522.**

| **Stamm (Plasmid)** | **GLA-Area %** | **Differenz zu CU522** |
|---|---|---|
| **CU522 ( - )** | 24,0 | - |
| **AX601 (pBDES6)** | 31,7 | + 32% |
| **AX604 (pBDES6)** | 29,3 | + 22% |

**Tabelle 2: Vergleich des Fettsäurespektrums (Hauptfettsäuren) der Tetrahymena pBDES6-Transformanten(AX601 und AX604) im Vergleich zumTetrahymena-Wildstamm (CU522) nach 50 h Kultivierung. Angegeben ist der prozentuale Anteil der Hauptfettsäuren am gesamt Fettsäurespektrum, sowie das Verhältniss der ungesättigten zu den gesättigten Haüptfettsäuren.**

| **Fettsäuren** | **Cu522** | **(-) AX601 (pBDES6)** | **AX604 (pBDES6)** |
|---|---|---|---|
| **C14:0** | 9,4 | 7,2 | 7,4 |
| **C14:1** | 2,8 | 3,5 | 3,1 |
| **C16:0** | 12,7 | 7,3 | 7,2 |
| **C16:1** | 4,5 | 6,4 | 6 |
| **C18:0** | 2,6 | - | 1,4 |
| **C18:1** | 9,5 | 3,9 | 4,8 |
| **C18:2** | 9,4 | 11,5 | 10,6 |
| **GLA (C18:3)** | 24 | 31,7 | 29,3 |
| **ungesättigt** | 50,2 | 57 | 53,8 |
| **gesättigt** | 24,7 | 14,5 | 16 |
| **ungesättigt/ gesättigt** | **2,03** | **3,93** | **3,36** |

Die Transformanten zeigten unter diesen Bedingungen einen um 22-32% erhöhten Anteil von GLA am Gesamtfettsäurespektrum gegenüber dem nicht-transformierten Stamm CU522 (Tabelle 1). Neben der Verschiebung des GLA-Anteils ist eine deutliche Verschiebung des Fettsäurespektrums zu einem höheren Gehalt an ungesättigten Fettsäuren auffällig. Das Verhältniss der ungesättigten zu den gesättigten (Haupt)Fettsäuren ist bei den Transformanten nahezu doppelt so hoch (Tabelle 2).

### BEISPIEL 9:

### Herstellung des delta-6-Desaturase Knock-out Konstruktes pgDES6::neo

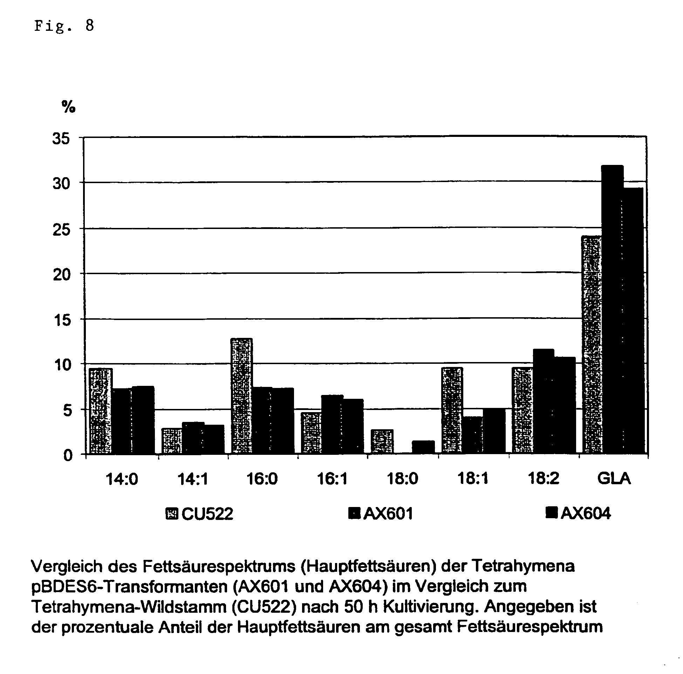

Für die Herstellung des Knock-out Konstruktes wurde in die genomische Sequenz der delta-6-Desaturase eine neo-Kassette aus dem Plasmid p4T2-1ΔH3 (Gaertig et al. (1994) Nucl. Acids Res. 22:5391-5398) inseriert. Dabei handelt es sich um das Neomycin-Resistenzgen unter der Kontrolle des Tetrahymena Histon H4-Promoters und der 3' flankierenden Sequenz des BTU2 Gens. Dieses Konstrukt vermittelt in Tetrahymena eine Resistenz gegenüber Paromomycin. Das Plasmid p4T2-1ΔH3 wurde mit Eco RV / Sma I geschnitten und das ca. 1,4 kb grosse Fragment der neo-Kassette wurde in das mit Eco RV geschnittene Plasmid pgDES6 in die genomische Sequenz der Tetrahymena delta-6-Desaturase ligiert (siehe Figur 8). Dadurch wurde das Plasmid pgDES6::neo erzeugt. Bei einer erfolgreichen Transformation wurde durch homologe Rekombination das Gen für die delta-6-Desaturase durch dieses Konstrukt ersetzt, wodurch eine Resistenz der Zellen gegenüber Paromomycin vermittelt wurde.

### BEISPIEL 10:

### Macronucleus-Transformation von Tetrahymena mit dem Desaturase Expressionskonstrukt pBDES6

Für eine Transformation wurden 5 x 10⁶ *Tetrahymena thermophila* Zellen (CU522) eingesetzt. Die Kultivierung der Zellen erfolgte in 50 ml SPPA-Medium bei 30 °C in einem 250 ml Erlenmeyerkolben auf einem Schüttler bei 150 RPM bis zu einer Zelldichte von ca. 3-5 x 10⁵ Zellen/ml. Die Zellen wurden durch Zentrifugation (1200 g) für 5 min pelletiert und das Zellpellet wurde in 50 ml 10 mM Tris-HCl (pH 7,5) resuspendiert und wie vorher zentrifugiert. Dieser Waschschritt wurde wiederholt und die Zellen in 10 mM Tris-HCl (pH 7,5, plus Antibiotika) bei einer Zelldichte von 3 x 10⁵ Zellen/ml resuspendiert, in einen 250 ml Erlenmeyerkolben überführt und für 16-20 h ohne Schütteln bei 30 °C inkubiert (Hungerphase). Nach der Hungerphase wurde erneut die Zellzahl bestimmt, wie oben zentrifugiert und die Zellen mit 10 mM Tris-HCl (pH 7,5) auf eine Konzentration von 5 x 10⁶ Zellen/ml eingestellt. Ein ml der Zellen wurde für die Transformation benutzt. Die Transformation erfolgte mittels Mikropartikelbeschuss (s.u.). Zur Regeneration wurden die Zellen in SSPA-Medium aufgenommen und bei 30 °C ohne Schütteln im Erlenmeyerkolben inkubiert. Nach 3 h wurde Pactitaxel^{®} in einer Endkonzentration von 20 µM zugegeben und die Zellen in Aliquots von 100 µl auf 96er Mikrotiterplatten überführt. Die Zellen wurden in einer feuchten, abgedunkelten Box bei 30 C inkubiert. Nach 2-3 Tagen konnten Paclitaxel resistente Klone identifiziert werden. Positive Klone wurden in frisches Medium mit 25 µM Paclitaxel überimpft. Durch Kultivierung der Zellen in steigender Paclitaxel-Konzentration (bis 80 µM) wurde ein komplettes "phenotypic assortment" (Gaertig & Kapler (1999)) erreicht.
Zur Analyse der Klone wurden ca. 4 ml Kulturen in SPPA mit Paclitaxel angezogen, DNA isoliert (Jacek Gaertig et al. (1994) PNAS 91:4549-4553) und durch PCR die in den BTU1-Locus integrierte DNA amplifiziert. Als Primer dienten die BTU1 spezifischen Primer BTU1-5'F (AAAAATAAAAAAGTTTGAAAAAAAACCTTC, ca 50 bp vor dem Startcodon, SEQ ID No.: 18) und BTU1-3'R (GTTTAGCTGACCGATTCAGTTC, 3 bp hinter dem Stopcodon, SEQ ID No.: 19). Die PCR-Produkte wurden ungeschnitten und mit Hind III oder Eco RV (pBDES6) bzw. Eco RI (pBDES9) geschnitten auf einem 1 %igen Agarosegel analysiert. Vollständiges "phenotypic assortment" wurde über RT-PCR mit den BTU1 spezifischen Primern (Gaertig & Kapler (1999)) überprüft.

### BEISPIEL 11:

### Micronucleus- und Macronucleus-Transformation von Tetrahymena mit dem Knock-out Konstrukt pgDES6::neo

Tetrahymena Stämme unterschiedlichen Paarungstyps (CU428 VII und B2086 II) wurden getrennt in SPPA-Medium bei 30 °C unter Schütteln (150 Upm) in einem Erlenmeyerkolben kultiviert. Bei einer Zelldichte von 3-5 x 10⁵ Zellen/ml wurden die Zellen 5 min bei Raumtemperatur zentrifugiert (1200 g). Die Zellen wurden dreimal mit 50 ml 10 mM Tris-HCl (pH 7,5) gewaschen und schließlich in 50 ml 10 mM Tris-HCl (pH 7,5) resuspendiert und mit Antibiotika-Lösung versetzt. Die Zellen wurden in einem Erlenmeyerkolben bei 30 °C ohne Schütteln inkubiert. Nach ca. 4 h wurden beide Kulturen erneut gezählt und mit 10 mM Tris-HCl (pH 7,5) auf 3 x 10⁵ Zellen/ml verdünnt für weitere 16-20 h bei 30 °C inkubiert. Nach dieser Hungerphase wurde von beiden Kulturen die gleiche (absolute) Zellzahl in einem 2-L Erlenmeyerkolben gemischt. Die Zellen wurden bei 30 °C inkubiert (Beginn der Konjugation) und nach 2 h wurde die Effizienz der Konjugation bestimmt. Für eine erfolgreiche Transformation sollten zu diesem Zeitpunkt ca. 30% der Zellen als Paare vorliegen.

Für die Micronucleus-Transformation wurden 3 h, 3,5 h, 4 h und 4,5 h nach Beginn der Konjugation jeweils 1 x 10⁷ konjugierende Zellen (5 x 10⁶ Paare) 5 min bei 1200 g zentrifugiert und das Zellpellet in 1 ml 10 mM Tris-HCl (pH 7,5) resuspendiert.
Für die Transformation der neuen Macronucleus-Anlagen wurden 11 h nach Beginn der Konjugation Zellen wie oben zentrifugiert und in Tris-HCl resuspendiert. Die Transformation erfolgte mittels Mikropartikelbeschuss (s.u.).
Für die Kultivierung der delta-6-Desaturase Knockout-Mutanten wurde 200µg/ml Borage-Öl (20-25% GLA; SIGMA) zum Medium zugegeben.

Durch Selektion auf Paromomycinresistenz konnten transformierte Zellen identifiziert werden. Bei der Transformation des Mikronucleus wurde 11 h nach Beginn der Konjugation Paromomycin (100 µg/ml Endkonzentration) zugegeben und die Zellen in Aliquots von 100 µl auf 96er Mikrotiterplatten verteilt. Die Zellen wurden in einer feuchten Box bei 30 °C inkubiert. Nach 2-3 Tagen konnten resistente Klone identifiziert werden. Echte Mikronucleus-Transformanten konnten anhand der Resistenz gegenüber 6-Methylpurin von Macronucleus-Transformanten unterschieden werden. Bei der Transformation des Macronucleus wurde ca. 4 h nach der Transformation Paromomycin (100 µg/ml Endkonzentration) zugegeben und die Zellen in Aliquots von 100 µl auf 96er Mikrotiterplatten verteilt. Die Zellen wurden in einer feuchten Box bei 30 °C inkubiert. Nach 2-3 Tagen konnten resistente Klone identifiziert werden. Positive Klone wurden in frisches Medium mit 120 µg/ml Paromomycin überimpft. Durch Kultivierung der Zellen in dieser hohen Paromomycin-Konzentration wurde nach einigen Generationen ein komplettes "phenotypic assortment" (Gaertig & Kapler (1999)) erreicht.
Durch Kreuzung der Micronucteus-Transformanten mit einem B*VI-Stamm konnten homozygote Knockout-Mutanten erzeugt werden (Bruns & Cassidy-Hanley, Methods in Cell Biology, Volume 62 (1999) 229-240).

### BEISPIEL 12:

### Biolistische Transformation (Mikropartikelbeschuss)

Die Transformation von *Tetrahymena thermophila* erfolgte mittels biolistischer Transformation, wie sie bei Bruns & Cassidy-Hanley (Methods in Cell Biology, Volume 62 (1999) 501-512); Gaertig et al. (1999) Nature Biotech. 17: 462-465) oder Cassidy-Hanley et al. ((1997) Genetics 146:135-147) beschrieben ist. Die Handhabung des Biolistic^{®} PDS-1000/He Particle Delivery System (BIO-RAD) ist detailiert im zugehörigen Handbuch beschrieben.
Für die Transformation werden 6 mg Goldpartikel (0,6 µm; BIO-RAD) mit 10 µg linearisierter Plasmid DNA beladen (Sanford et al. (1991) Biotechniques 3:3-16; Bruns & Cassidy-Hanley (1999) Methods in Cell Biology, Volume 62: 501-512).
Vorbereitung der Goldpartikel: 60 mg der 0,6 µm Goldpartikel (Biorad) wurden in 1 ml Ethanol resuspendiert. Dazu wurden die Partikel 3 mal für je 1-2 min auf einem Vortex kräftig gemixt. Anschliessend wurden die Partikel für 1 min zentrifugiert (10000 g) und der Überstand vorsichtig mit einer Pipette abgenommen. Die Goldpartikel wurden in 1 ml sterilem Wasser resuspendiert und wie oben zentrifugiert. Dieser Waschschritt wurde einmal wiederholt, die Partikel in 1 ml 50%igem Glycerol resuspendiert und in Aliquots zu 100 µl bei -20 °C gelagert.

Vorbereiten der Transformation: Die Macrocarrierhalter, Macrocarrier und Stopscreens wurden für mehrere Stunden in 100% Ethanol, die Rupture Disks in Isopropanol gelagert. Ein Macrocarrier wurde anschließend in den Macrocarrier-Halter eingesetzt und an der Luft getrocknet.

Beladung der Goldpartikel mit DNA: Alle Arbeiten erfolgten bei 4 °C. Goldpartikel, vorbereiteter Vektor, 2,5 M CaCl₂, 1 M Spermidine, 70% und 100% Ethanol wurdenauf Eis gekühlt. 10 µl der linearisierten Vektor-DNA (1 µg/ml) wurden zu 100 µl vorbereiteten Goldpartikeln gegeben und für 10 sec vorsichtig gevortext. Anschliessend wurden erst 100 µl 2,5 M CaCl₂ zugegeben, 10 sec gevortext und dann 40 µl 1M Spermidine zugegeben und 10 min vorsichtig gevortext. Nach Zugabe von 200 µl 70%igem Ethanol wurden die Partikel für 1 min gevortext und dann 1 min bei 10000 g zentrifugiert. Das Pellet wurde in 20 µl 100%igem Ethanol resuspendiert, zentrifugiert und dann in 35 µl 100%igem Ethanol resuspendiert.
Die so vorbereiteten Partikel wurden vorsichtig mit einer Pipette auf das Zentrum eines Macrocarriers gegeben. Der Macrocarrierer wurde anschliessend bis zur Transformation in einer Box mit hygroskopischen Silicagel gelagert.

Transformation: Ein ml der vorbereiteten Zellen (siehe oben) wurde in die Mitte eines mit 10 mM Tris-HCl (pH 7,5) angefeuchteten Rundfilters in einer Petrischale gegeben und in die unterste Einschubleiste der Transformationskammer des Biolistic^{®} PDS-1000/He Particle Delivery Systems eingesetzt. Die Transformation erfolgte mit den vorbereiteten Goldpartikeln bei einem Druck von 900 psi (zwei 450 psi Rupture Disks) und einem Vacuum von 27 inches Hg in der Transformationskammer. Anschliessend wurden die Zellen sofort in einen Erlenmeyerkolben mit 50 ml SPPA-Medium überführt und bei 30 °C ohne Schütteln inkubiert.

## Patentansprüche

1. Nukleinsäure kodierend für delta-6-Desaturase aus Tetrahymena mit einer Aminosäuresequenz gemäß SEQ ID NO: 2 und Teile davon mit mindestens 8 Nukleotiden, die für ein Polypeptid kodieren, das delta-6-Desaturase-Aktivität aufweist.

2. Nukleinsäure nach Anspruch 1, **dadurch gekennzeichnet, daß** die Nukleinsäure aus einem Ciliaten erhalten wird.

3. Nukleinsäure nach Anspruch 1 oder 2, **dadurch gekennzeichnet, daß** die Nukleinsäure aus *Tetrahymena thermophila* erhalten wird.

4. Nukleinsäure nach Anspruch 1 - 3, **dadurch gekennzeichnet, daß** die Nukleinsäure eine DNA oder RNA, vorzugsweise eine doppelsträngige DNA, ist.

5. Nukleinsäure nach einem der Ansprüche 1 bis 4, **dadurch gekennzeichnet, daß** die Nukleinsäure eine DNA mit einer Nukleinsäuresequenz gemäß SEQ ID No.: 1 von Position 33 bis Position 1091 ist.

6. Nukleinsäure nach einem der Ansprüche 1 bis 5, **dadurch gekennzeichnet, daß** die Nukleinsäure eine oder mehrere nicht kodierende Sequenzen enthält.

7. Eine isolierte Nukleinsäure nach einem der Ansprüche 1 bis 6 gemäß SEQ ID NO: 3 und Teile davon mit mindestens 8 Nukleotiden, die für ein Polypeptid kodieren, das delta-6-Desaturase-Aktivität aufweist.

8. Nukleinsäuren nach einem der Ansprüche 1 - 7, **dadurch gekennzeichnet, daß** die Nukleinsäure in einem Vektor, vorzugsweise in einem Expressionsvektor, enthalten ist.

9. Expressionvektoren nach Anspruch 8, **dadurch gekennzeichnet**, das die Nukleinsäure in funktioneller Kombination zu einem konstitutiven und / oder induzierbaren Promoter und optional einem Terminationssignal steht.

10. Verfahren zur Herstellung einer Nukleinsäure nach einem der Ansprüche 1-7, **dadurch gekennzeichnet, daß** die Nukleinsäure chemisch synthetisiert oder anhand einer Sonde aus einer Genbank isoliert wird.

11. Polypeptid mit einer Aminosäuresequenz gemäß SEQ ID NO: 2 und Teile davon mit mindestens 12 Aminosäuren, die delta-6-Desaturase-Aktivität aufweisen.

12. Verfahren zur Herstellung eines Polypeptids gemäß Anspruch 11, **dadurch gekennzeichnet, daß** eine Nukleinsäure gemäß einem der Ansprüche 1-7 in einem geeigneten Expressionssystem oder Wirtsorganismus exprimiert wird.

13. Spezifischer Antikörper gegen ein Polypeptid mit einer Aminosäuresequenz gemäß SEQ ID NO: 2.

14. Transgener nicht-humaner Organismus enthaltend eine Nukleinsäure nach einem der Ansprüche 1 bis 10.

15. Nukleinsäure nach Anspruch 1, **dadurch gekennzeichnet, dass** die Nukleinsäure SEQ ID NO: 1 umfasst.

16. Nukleinsäure nach Anspruch 7, **dadurch gekennzeichnet, dass** die Nukleinsäure SEQ ID NO: 3 umfasst.

17. Nukleinsäure nach Anspruch 1 oder 7, **dadurch gekennzeichnet, dass** die Teile mindestens 15, vorzugsweise 20 Nukleotide umfassen.

18. Nukleinsäure nach Anspruch 1 oder 7, **dadurch gekennzeichnet, dass** die Teile mindestens 100 Nukleotide umfassen.

## Claims

1. A nucleic acid which codes for delta-6-desaturase from Tetrahymena having an amino acid sequence as shown in SEQ ID No.: 2 and parts thereof comprising at least 8 nucleotides which code for a polypeptide which has delta-6-desaturase activity.

2. The nucleic acid according to claim 1, **characterized in that** the nucleic acid is obtained from a ciliate.

3. The nucleic acid according to claim 1 or 2, **characterized in that** the nucleic acid is obtained from *Tetrahymena thermophila.*

4. The nucleic acid according to claims 1 to 3, **characterized in that** the nucleic acid is a DNA or RNA, preferably a double-stranded DNA.

5. The nucleic acid according to any one of claims 1 to 4, **characterized in that** the nucleic acid is a DNA having a nucleic acid sequence as shown in SEQ ID No.: 1 from position 33 to position 1091.

6. The nucleic acid according to any one of claims 1 to 5, **characterized in that** the nucleic acid contains one or more non-coding sequences.

7. An isolated nucleic acid according to any one of claims 1 to 6 as shown in SEQ ID No.: 3 and parts thereof comprising at least 8 nucleotides which code for a polypeptide which has delta-6-desaturase activity.

8. Nucleic acids according to any one of claims 1 to 7, **characterized in that** the nucleic acid is contained in a vector, preferably in an expression vector.

9. Expression vectors according to claim 8, **characterized in that** the nucleic acid is in functional combination with a constitutive and/or inducible promoter and optionally a termination signal.

10. A process for producing a nucleic acid according to any one of claims 1 to 7, **characterized in that** the nucleic acid is chemically synthesised or is isolated from a gene library using a probe.

11. A polypeptide having an amino acid sequence as shown in SEQ ID No.: 2 and parts thereof comprising at least 12 amino acids which have delta-6-desaturase activity.

12. A process for producing a polypeptide according to claim 11, **characterized in that** a nucleic acid according to any one of claims 1 to 7 is expressed in a suitable expression system or host organism.

13. A specific antibody against a polypeptide having an amino acid sequence as shown in SEQ ID No.: 2.

14. A transgenic non-human organism containing a nucleic acid according to any one of claims 1 to 10.

15. The nucleic acid according to claim 1, **characterized in that** the nucleic acid comprises SEQ ID No.: 1.

16. The nucleic acid according to claim 7, **characterized in that** the nucleic acid comprises SEQ ID No.: 3.

17. The nucleic acid according to claim 1 or 7, **characterized in that** the parts comprise at least 15, preferably 20 nucleotides.

18. The nucleic acid according to claim 1 or 7, **characterized in that** the parts comprise at least 100 nucleotides.

## Revendications

1. Acide nucléique codant pour delta-6-désaturase issue de Tetrahymena avec une séquence d'acides aminés conforme SEQ ID NO: 2 et comportant des fragments avec au moins 8 nucléotides codant pour un polypeptide qui montre une activité de la delta-6-désaturase.

2. Acide nucléique selon revendication 1, **caractérisé en ce que**, l'acide nucléique est issue d'un cilié.

3. Acide nucléique selon revendication 1 ou 2, **caractérisé en ce que**, l'acide nucléique est issue de Tetrahymena thermophila.

4. Acide nucléique selon revendication 1-3, **caractérisé en ce que**, l'acide nucléique est une ADN ou bien ARN, de préférence une ADN en double hélice.

5. Acide nucléique selon l'une des revendications 1 jusqu'à 4, **caractérisé en ce que**, l'acide nucléique est une ADN comprenant une séquence d'acide nucléique conforme SEQ ID No : 1, de la position 33 jusqu'à la position 1091.

6. Acide nucléique selon l'une des revendications 1 jusqu'à 5, **caractérisé en ce que**, l'acide nucléique comprend une ou plusieurs séquences non codantes.

7. Un acide nucléique isolé selon l'une des revendications 1 à 6 conforme SEQ ID NO : 3 et des parties de ce derniers avec au moins 8 nucléotides codant pour un polypeptide qui montre une activité de la delta-6-désaturase.

8. Acide nucléique selon l'une des revendications 1 - 7, **caractérisé en ce que**, l'acide nucléique est contenu dans un vecteur, de préférence dans un vecteur d'expression.

9. Vecteur d'expression selon revendication 8, **caractérisé en ce que**, l'acide nucléique représente une combinaison fonctionnelle avec un promoteur constitutif et/ou inductif et optionnellement un signal de terminaison.

10. Procédure de production d'un acide nucléique selon l'une des revendications 1-7, **caractérisé en ce que**, l'acide nucléique est synthétisé chimiquement ou bien isolé d'une banque de gènes à l'aide d'une sonde.

11. Polypeptide avec une séquence d'acides aminés conforme SEQ ID NO : 2 et des fragments avec au moins 12 acides aminés, qui montrent une activité de la delta-6-désaturase.

12. Procédure de production d'un polypeptide selon revendication 11, **caractérisé en ce que**, un acide nucléique selon l'une des revendications 1-7 est exprimé dans un système d'expression approprié ou bien dans un organisme hôte.

13. Anticorps spécifique contre un polypeptide avec une séquence d'acides aminés conforme SEQ ID NO : 2.

14. Organisme transgénique non-humain comportant un acide nucléique selon l'une des revendications 1 à 10.

15. Acide nucléique selon revendication 1, **caractérisé en ce que**, l'acide nucléique comporte SEQ ID NO : 1.

16. Acide nucléique selon revendication 7, **caractérisé en ce que**, l'acide nucléique comporte SEQ ID NO : 3.

17. Acide nucléique selon revendication 1 ou 7, **caractérisé en ce que**, les fragments comportent au moins 15, de préférence 20 nucléotides.

18. Acide nucléique selon revendication 1 ou 7, **caractérisé en ce que**, les fragments comportent au moins 100 nucléotides.
